# EUROPEAN PATENT APPLICATION

(11) **EP 4 624 487 A1**
(43) Date of publication of application: **01.10.2025**
(21) Application number: 23894637.0
(22) Date of filing: 22.11.2023
(51) Int. Cl.: C07K 14/47, A61K 31/7105, A61K 39/00, A61K 48/00, A61P 43/00, C07K 7/08, C07K 14/525, C07K 16/18, C07K 16/24, C12N 15/11

(54) **PRODUCTION OF ANTIBODIES AGAINST PROTEIN**

(30) Priority: 24.11.2022 JP 2022187508
(71) Applicant: Tokushima University, Tokushima-shi, Tokushima 770-8501 (JP)
(72) Inventor: ISHIDA, Tatsuhiro, Tokushima-shi, Tokushima 770-8501 (JP); OHMOTO, Yasukazu, Tokushima-shi, Tokushima 770-8501 (JP); ANDO, Hidenori, Tokushima-shi, Tokushima 770-8501 (JP); TANAKA, Michinori, Osaka-shi, Osaka 540-0021 (JP); SASAHARA, Kenichi, Osaka-shi, Osaka 540-0021 (JP)
(74) Representative: dompatent von Kreisler Selting Werner - Partnerschaft von Patent- und Rechtsanwälten mbB
(86) International application number: PCT/JP2023/042024
(87) International publication number: WO 2024/111633

(57) **Abstract**

The present invention provides Methods for inducing antibodies against target proteins by using a peptide as an antigen.

The invention relates to a method for preparing a partial peptide within a target protein, which induces an antibody specific to the target protein, which comprises: 1) providing a plurality of partial peptides of 5-30 amino acid residues, 2) making a mixture containing the partial peptides obtained, 3) immunizing non-human mammals by use of the obtained mixture, 4) determining whether the mixture induces any antibody specific to the target protein by the immunization; and 5) identifying the partial peptide that induces the specific antibody from the mixture that has been determined to induce the antibody.

## Description

### TECHNICAL FIELD

The present application claims the priority under the Paris Convention with respect to the Japanese Patent Application No. 2022-187508 filed on November 24, 2022, which is incorporated herein by reference in its entirety.

The present invention generally relates to a method for preparing antibodies to proteins, and specifically antibodies against epitopes (antigenic peptides) within proteins, that is, peptide antibodies. More specifically, the present invention relates to a method for preparing a partial peptide of a protein that has a physiological activity, which can induce an antibody against the protein, the partial peptide itself, and a vaccine comprising the partial peptide.

### BACKGROUND TECHNOLOGY

To prepare a peptide antibody, it is necessary to synthesize a peptide. In 1959, Dr. Robert Merrifield from the United States discovered the solid-phase peptide synthesis method and, after further research, published the method in the Journal of the American Chemical Society in 1963. Subsequently, they succeeded in being the first in the world to produce bradykinin, angiotensin, insulin, and the like (Wikipedia).

In the preparation of a peptide antibody, a peptide should be selected, which contains an epitope having an amino acid sequence providing any stronger antigenicity within the amino acid sequence of the protein. At the time before 1980 when low molecular weight peptide hormones were abundant, peptide synthesis was performed for the whole region if possible, or if it is difficult, the synthesis was performed by dividing the whole region into the N-terminal region and the C-terminal region. In particular, the mature type of the N-terminal region peptide cleaved from its precursor was selected. In the beginning, antibodies against low molecular weight peptide hormones such as insulin and glucagon, which were extracted and purified from biological components, were prepared, and a radioimmunoassay (RIA) using these antibodies to measure the low molecular weight hormones was reported (Non-Patent Document 1). Further, the amino acid sequences of the purified peptide hormones have been determined, and advances in peptide synthesis technology has facilitated to chemically synthesize either the whole region of the peptide hormone, or its N-terminal region or C-terminal region as an immunogenic antigen necessary for antibody preparations. To enhance the antigenicity of the synthesized peptides, carrier proteins such as KLH and BSA were coupled. It has been reported that the coupled materials were immunized in rats and rabbits to provide antisera so that radioimmunoassay was established (Non-Patent Document 2).

Around 1980, due to the development of genetic engineering technology, amino acid sequences of many physiologically active substances have been reported. In particular, there have been many reports on the complete amino acid sequences of cytokines. In 1980 when the complete amino acid sequence of the interferon was reported (Non-Patent Document 3), the N-terminal and C-terminal peptides thereof synthesized were chemically modified to a carrier protein, and those materials were administered to rabbits as antigens to prepare anti-peptide sera. Among the multiple anti-peptide sera prepared in this process, high-titer anti-peptide sera with high cross-reactivity to the derived protein were selected rather than the administered peptide, so as to establish a highly sensitive measurement system for the derived protein (Non-Patent Document 4). Additionally, the nucleic acid sequences of retroviruses (HTLV-1) have been reported (Non-Patent Document 5), and antibodies to the N-terminal and C-terminal peptides were utilized for the identification of the gene products (proteins) of the open reading frame of the HTLV-1 (Non-Patent Document 6, 7). During this period (the 1980s), peptide synthesis has changed from liquid-phase methods to solid-phase methods, and peptide synthesis using peptide synthesizers has been widely available to the public (Non-Patent Document 8).

In the 1980s when genetic engineering technology was widely available, amino acid sequences of macromolecular cytokines and viruses were identified by companies and research institutions, and in order to select peptide regions ranging from 10 to 30 amino acids from the amino acid sequences, the hydrophilic and hydrophobic regions of proteins and their secondary structures have been inferred (Non-Patent Document 9, 10). For example, antibodies to peptides selected as antigenic regions within the TNFα protein have been reported (Non-Patent Document 11). In addition, during the 1980s, genetic recombinant technology enabled the large-scale production of proteins having physiological activities that were close to natural proteins in Escherichia coli and animal cells. As a result, for highsensitivity measurement systems such as RIA and ELISA, recombinant proteins (the whole region) were then immunized in mice to prepare monoclonal antibodies (Non-Patent Document 12), and peptide antibodies were no longer the first choice. However, since the preparation of recombinant proteins involves steps such as gene cloning, expression, culture and purification, thereby requiring time and cost, peptide antibodies, which can be relatively easily prepared, have been effective tools in tissue staining and Western blot analysis for cell surface markers and the like (Non-Patent Document 13). Moreover, phosphorylated peptide antibodies that recognize modified proteins such as phosphorylated proteins have been important tools in research for signal transduction.

In the 2000s, the epitope databases IEDB (Non-Patent Document 14) and SYFPEITHI (Non-Patent Document 15) have been available in public. The IEDB database is an epitope analysis tool developed with funding from the NIH in the United States, reflecting journal articles published since 1960 (over 95% or more of the relevant published literature) (Non-Patent Document 14).

The epitope analysis includes the prediction for B cell epitope-MHC binding and the prediction for T cell epitope-MHC binding, with the former including seven types of predictions: 1) Bepipred linear epitope prediction 2.0, 2) Bepipred linear epitope prediction, 3) Chou & Fasman beta-turn prediction, 4) Emini surface accessibility prediction, 5) Karplus & Schulz flexibility prediction, 6) Kolaskar & Tongaonkar antigenicity, and 7) Parker's hydrophilicity prediction, wherein, for example, the sequence of a specific protein is attached to 3) Chou & Fasman beta-turn prediction to graph the hydrophilic and hydrophobic regions of the protein, allowing for the selection of 10 to 30 peptide sequences from the indicated regions. According to the latter, it is possible to identify candidate peptides that can bind to the MHC class I molecule and the MHC class II molecule. For example, when the sequence of a specific protein is attached to 1), the peptides that are presented on the MHC class I molecule (9 killer peptides) are provided, and when the sequence is attached to 2), the peptides that are presented on the MHC class II molecule (about 15 helper peptides) are provided. Since numerous peptides are provided as killer and helper peptides, it is necessary to use peripheral blood, cancer cells and the like for screening for peptides that activate T cells.

Since a lot of peptides are not immunogenic on their own, the induction of peptide antibodies requires that, after peptide synthesis, the peptides should be conjugated to a carrier protein (carrier(s)) to ensure a robust immune response with high antibody titers. In the case of animals, bovine serum albumin, Keyhole Limpet Hemocyanin (KLH), and ovalbumin can be used as carriers (Non-Patent Document 15, 16, 17, 18, 19). In the case of humans, the detoxified diphtheria toxin variant CRM197 (CRM, Cross-Reaction Material) is used as a carrier in pneumococcal vaccines, although examples are few (Non-Patent Document 20).

The selected peptide and carrier can be conjugated using bifunctional reagents such as glutaraldehyde, carbodiimide, N-succinimidyl 3-(2-pyridyldithio)propionate, and 3-maleimido benzoic acid N-hydroxysuccinimide ester. The immunization is conducted by mixing the peptide-carrier conjugate and the adjuvant (various types of adjuvants are also used), and administering the same subcutaneously to the selected animals (most often, mice or rabbits). After repeated administration, it is necessary to screen the antibody titer (Non-Patent Document 21, 22). Since the epitopes are usually 5-8 amino acids in length, peptides of 15-20 amino acid residues, which are commonly used in the immunization, may elicit antibody responses that recognize multiple epitopes (Non-Patent Document 21, 22). The use of shorter peptides may help to avoid this, but by doing so, there is also a possibility that the total potency of the antibodies could be decreased, thereby reducing the immunogenicity.

On the other hand, when peptide antibodies are induced in humans, it is necessary to administer a cancer vaccine on a trial basis, which involves a gradual and long-term treatment, while taking account of the patients' quality of life (Non-Patent Document 23). Without using highly antigenic carrier proteins (carriers), only peptides with adjuvants that can be administered to humans or liposomeencapsulated peptides are used to conduct subcutaneous or intramuscular injections given bi-weekly or bi-monthly thereby inducing peptide antibodies. Administration frequency of the cancer vaccine and other parameters should be controlled by collecting fractionized bloods during the administration period, and monitoring the concentrations of neutralizing antibodies and binding antibodies in the collected sera.

### CITATION LIST

### NON-PATENT DOCUMENTS

Non-Patent Document 1: Trenkle A., Radioimmunoassay of plasma hormones: review of plasma insulin in ruminants., J Dairy Sci. 1972 Aug; 55(8):1200-11.
Non-Patent Document 2: Nishino T, Kodaira T, Shin S, Imagawa K, Shima K, Yanaihara C, Yanaihara N., Glucagon radioimmunoassay with use of antiserum to glucagon C-terminal fragment., Clin Chem. 1981 Oct; 27(10):1690-7.
Non-Patent Document 3: T Taniguchi, N Mantei, M Schwarzstein, S Nagata, M Muramatsu, C Weissmann, Human leukocyte and fibroblast interferons are structurally related, Nature 1980 Jun 19;285(5766):547-9.
Non-Patent Document 4: Shimizu F, Ohmoto Y, Imagawa K. Production of anti-IFN-beta sera with chemically synthetic IFN-beta peptide fragment (1 - 13)., Biochem Biophys Res Commun. 1981 Dec 31; 103(4):1149-56.
Non-Patent Document 5: M Yoshida, I Miyoshi, and Y Hinuma, Isolation and characterization of retrovirus from cell lines of human adult T-cell leukemia and its implication in the disease., Proc Natl Acad Sci U S A. 1982 Mar; 79(6): 2031-2035.
Non-Patent Document 6: Hattori S, Kiyokawa T, Imagawa K, Shimizu F, Hashimura E, Seiki M, Yoshida M., Identification of gag and env gene products of human T-cell leukemia virus (HTLV)., Virology. 1984 Jul 30; 136(2):338-47.
Non-Patent Document 7: Kiyokawa T, Seiki M, Iwashita S, Imagawa K, Shimizu F, Yoshida M. p27x-III and p21x-III, proteins encoded by the pX sequence of human T-cell leukemia virus type I., Proc Natl Acad Sci U S A. 1985 Dec;82(24):8359-63.
Non-Patent Document 8: G H Fisher, K Folkers, B Pernow, C Y Bowers, Synthesis and some biological activities of the tyrosine-8 analog of substance P J Med Chem. 1976 Feb; 19(2):325-8.
Non-Patent Document 9: Chou PY, Fasman GD., Empirical predictions of protein conformation., Annu Rev Biochem. 1978
Non-Patent Document 10: Hopp T. P., Woods K. R., Prediction of protein antigenic determinants from amino acid sequences, Proc. Nati. Acad. Sci. USA Vol. 78, No. 6, pp. 3824-3828, June 1981
Non-Patent Document 11: S H Socher 1, M W Riemen, D Martinez, A Friedman, J Tai, J C Quintero, V Garsky, A Oliff, Antibodies against amino acids 1-15 of tumor necrosis factor block its binding to cell-surface receptor, Proc Natl Acad Sci U S A. 1987 Dec; 84(24):8829-33.
Non-Patent Document 12: Tracey KJ, Fong Y, Hesse DG, Manogue KR, Lee AT, Kuo GC, Lowry SF, Cerami A., Anticachectin/TNF monoclonal antibodies prevent septic shock during lethal bacteraemia., Nature. 1987 Dec 17-23; 330(6149):662-4.
Non-Patent Document 13: H Clevers 1, S Dunlap, C Terhorst, The transmembrane orientation of the epsilon chain of the TcR/CD3 complex, Eur J Immunol. 1988 May; 18(5):705-10.
Non-Patent Document 14: Randi Vita, James A Overton, Jason A Greenbaum, Julia Ponomarenko, Jason D Clark, Jason R Cantrell, Daniel K Wheeler, Joseph L Gabbard, Deborah Hix, Alessandro Sette, Bjoern Peters, The immune epitope database (IEDB) 3.0, Nucleic Acids Res. 2015 Jan; 43(Database issue): D405-12.
Non-Patent Document 15: Rammensee HG, Bachmann J, Emmerich NPN, Bachor OA, Stevanovic S., SYFPEITHI: database for MHC ligands and peptide motifs. , Immunogenetics (1999) 50:213-9. doi: 10.1007/s002510050595
Non-Patent Document 16: Zarei O., Irajian G.R., Zarnani A.H., Chamani-Tabriz L., Emami S., Jeddi-Tehrani M., Rabbani H., Peptide-based polyclonal antibody production against p110 protein of mycoplasma genitalium., Avicenna J. Med. Biotechnol. 2011; 3: 79-85.
Non-Patent Document 17: Trier N.H., Houen G., Peptide antibodies in clinical laboratory diagnostics., Adv. Clin. Chem. 2017; 81: 43-96.
Non-Patent Document 18: Houen G., Jakobsen M.H., Svaerke C., Koch C., Barkholt V., Conjugation to preadsorbed preactivated proteins and efficient generation of anti peptide antibodies., J. Immunol. Methods. 1997; 206: 125-134. doi: 10.1016/S0022-1759(97)00097-5.
Non-Patent Document 19: Houen G., Jensen O.M., Conjugation to preactivated proteins using divinylsulfone and iodoacetic acid., J. Immunol. Methods. 1995; 181: 187-200. doi: 10.1016/0022-1759(94)00345-W.
Non-Patent Document 20: Hurdayal R., Achilonu I., Choveaux D., Coetzer T.H., Dean Goldring J.P., Anti-peptide antibodies differentiate between plasmodial lactate dehydrogenases., Peptides. 2010; 31: 525-532.
Non-Patent Document 21: Package Insert
Non-Patent Document 22: Pihl T.H., Illigen K.E., Houen G., Polyclonal peptide antisera., Methods Mol. Biol. 2015; 1348: 103-107.
Non-Patent Document 23: Trier N.H., Mortensen A., Schiolborg A., Friis T., Production and screening of monoclonal peptide antibodies., Methods Mol. Biol. 2015; 1348: 109-126.
Non-Patent Document 24: A new peptide vaccine OCV-501: in vitro pharmacology and phase 1 study in patients with acute myeloid leukemia, Yukio Kobayashi, Toru Sakura, Shuichi Miyawaki, Kazuyuki Toga, Shinji Sogo, Yuji Heike, Cancer Immunol Immunother. 2017; 66(7): 851-863. Published online 2017 Mar 20.

### SUMMARY OF THE INVENTION

### PROBLEMS TO BE SOLVED BY THE INVENTION

Low molecular weight peptides are rapidly degraded by serum proteases upon entering into the body, and are excreted in the urine. Low molecular weight peptides themselves typically do not have any ability to induce antibodies, and in order to induce peptide antibodies against low molecular weight peptides, it is necessary to chemically conjugate the low molecular weight peptides to carrier proteins such as KLH or CRM197. The inventors of the present application have chemically synthesized peptide fragments within the polymeric bioactive proteins and discovered that some peptides, which would normally lack an antibody-inducing ability, possess the ability to induce antibodies.

### MEANS TO SOLVE PROBLEMS

In view of these circumstances, the present invention aims to provide a novel peptide antigen which is a partial peptide from a target protein recognized by an antibody medicament that has been approved and marketed as a therapeutic drug, which has been checked to induce an antibody similar to the antibody medicament by administering the partial peptide.

Specifically, in order to solve the above-mentioned problems, the inventors of the present application conducted various studies which comprise synthesizing five peptides from human amyloid β-protein (42aa), forty-six peptides from human TNFα (157aa), twenty-three peptides from mouse PD-1 (288aa, extracellular domain 169aa), nineteen peptides from human PD-1, and twenty-nine peptides from human PD-L1, administering these peptides mixed with an adjuvant to rabbits, and checking the induction of three, ten, fourteen, four, and twelve peptide antibodies, respectively, as well as checking that those peptide antibodies also bind to the target proteins, thereby completing the present invention.

Therefore, the present invention encompasses the aspects as follows.

### <Methods for preparing partial peptides having abilities to induce antibodies against target proteins>

[1] A method for preparing a partial peptide within a target protein, which induces an antibody specific to the target protein, which comprises:
   1) providing a plurality of partial peptides of 5-30 amino acid residues within the amino acid sequence of the target protein, wherein each partial peptide may overlap with an adjacent different partial peptide by a specific number of amino acid residues at its N-terminus or C-terminus, and wherein the plurality of the partial peptides covers the entire region of the amino acid sequence of the target protein;
   2) making a mixture containing the 4 to 12 partial peptides obtained;
   3) immunizing non-human mammals by use of the obtained mixture;
   4) determining whether the mixture induces any antibody specific to the target protein by the immunization; and
   5) identifying the partial peptide that induces the specific antibody from the mixture that has been determined to induce the antibody.
[2] The method according to [1], which further comprises;
   6) a step of adding an additional sequence to the identified partial peptide.
[3] The method according to [2], wherein step 6) comprises adding the additional sequence to make the identified partial peptide 50 amino acids in length or less.
[4] The method according to [3], wherein step 6) comprises adding the additional sequence to make the partial peptide 30 amino acids in length.
[5] The method according to [1], wherein the non-human mammal is a mouse, rabbit, or monkey in step 3).
[6] The method according to [1], wherein the partial peptide contained in the mixture is not chemically bonded to any carrier protein.

### <Partial peptides having abilities to induce antibodies against target proteins>

[7] A partial peptide within a target protein identified by the method for preparation according to [1], which induces an antibody specific to the target protein, or a mutant partial peptide having one or several substitutions, deletions, or additions of amino acid residues within the amino acid sequence of the partial peptide, and having an equivalent activity to the partial peptide.
[8] The partial peptide or the mutant partial peptide according to [7], wherein the partial peptide induces an antibody specifically binding to human TNFα, and has an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCQRETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), and wherein the mutant partial peptide has one or several substitutions, deletions, or additions of amino acid residues within the amino acid sequence of the partial peptide, and has an equivalent activity to the partial peptide.
[9] The partial peptide or the mutant partial peptide according to [7], wherein the partial peptide induces an antibody specifically binding to human β-amyloid peptide, and has an amino acid sequence of SGYEVHHQKLVFFA (SEQ ID NO: 50), and wherein the mutant partial peptide has one or several substitutions, deletions, or additions of amino acid residues within the amino acid sequence of the partial peptide, and has an equivalent activity to the partial peptide.
[10] The partial peptide or the mutant partial peptide according to [7], wherein the partial peptide induces an antibody specifically binding to human PD-L1, and has an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), and wherein the mutant partial peptide has one or several substitutions, deletions, or additions of amino acid residues within the amino acid sequence of the partial peptide, and has an equivalent activity to the partial peptide.
[11] The partial peptide or the mutant partial peptide according to [7], wherein the partial peptide induces an antibody specifically binding to human PD-1, and has an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), and wherein the mutant partial peptide has one or several substitutions, deletions, or additions of amino acid residues within the amino acid sequence of the partial peptide, and has an equivalent activity to the partial peptide.
[12] The partial peptide or the mutant partial peptide according to [7], wherein the partial peptide induces an antibody specifically binding to mPD-1, and has an amino acid sequence selected from GWLLEVPNGPWRSLT (SEQ ID NO: 55), ATFTCSLSNWSEDLM (SEQ ID NO: 56), KQAAFCNGLSQPVQD (SEQ ID NO: 57), and FHMNILDTRRNDSGI (SEQ ID NO: 58), and wherein the mutant partial peptide has one or several substitutions, deletions, or additions of amino acid residues within the amino acid sequence of the partial peptide, and has an equivalent activity to the partial peptide.

### <Peptide vaccines>

[13] A vaccine for preventing and/or treating a disease related to a target protein, which comprises a partial peptide within the target protein identified by the method for preparation according to [1], which induces an antibody specific to the target protein, or a mutant partial peptide.
[14] The vaccine according to [13], which comprises a plurality of the partial peptides and/or the mutant partial peptides.
[15] The vaccine according to [13] for preventing and/or treating a disease related to human TNFα, wherein the partial peptide induces an antibody specifically binding to human TNFα, and has an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCQRETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), and wherein the mutant partial peptide has one or several substitutions, deletions, or additions of amino acid residues within the amino acid sequence of the partial peptide, and has an equivalent activity to the partial peptide.
[16] The vaccine according to [13] for preventing and/or treating a disease related to human β-amyloid peptide, wherein the partial peptide induces an antibody specifically binding to human β-amyloid peptide, and has an amino acid sequence SGYEVHHQKLVFFA (SEQ ID NO: 50), and wherein the mutant partial peptide has one or several substitutions, deletions, or additions of amino acid residues within the amino acid sequence of the partial peptide, and has an equivalent activity to the partial peptide.
[17] The vaccine according to [13] for preventing and/or treating a disease related to human PD-L1 protein, wherein the partial peptide induces an antibody specifically binding to human PD-L1 protein, and has an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), and wherein the mutant partial peptide has one or several substitutions, deletions, or additions of amino acid residues within the amino acid sequence of the partial peptide, and has an equivalent activity to the partial peptide.
[18] The vaccine according to [13] for preventing and/or treating a disease related to human PD-1 protein, wherein the partial peptide induces an antibody specifically binding to human PD-1 protein, and has an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), and wherein the mutant partial peptide has one or several substitutions, deletions, or additions of amino acid residues within the amino acid sequence of the partial peptide, and has an equivalent activity to the partial peptide.

### <RNA vaccines>

[19] A vaccine for preventing and/or treating a disease related to a target protein, which comprises mRNA coding for a partial peptide within the target protein identified by the method for preparation according to [1], which induces an antibody specific to the target protein, or a mutant partial peptide.
[20] The vaccine according to [19], which comprises a plurality of the mRNAs coding for the partial peptides and/or the mutant partial peptides.
[21] The vaccine according to [19] for preventing and/or treating a disease related to human TNFα, wherein the mRNA codes for a partial peptide inducing an antibody specifically binding to human TNFα, and having an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSA1KSPCQRETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), or a mutant partial peptide having one or several substitutions, deletions, or additions of amino acid residues within the amino acid sequence of the partial peptide, and having an equivalent activity to the partial peptide.
[22] The vaccine according to [21], wherein the mRNA is selected from GGGCAGCUCCAGUGGCUGAACCGCCGGGCCAAUGCCCUCCUGGCC (SEQ ID NO: 59), CCCUGCCAGAGGGAGACCCCAGAGGGGGCUGAGGCCAAGCCCUGG (SEQ ID NO: 60), CUCCAGUGGCUGAACCGCCGGGCCAAUGCCCUCCUGGCCAAUGGC (SEQ ID NO: 61), and GACUUUGCCGAGUCUGGGCAGGUCUACUUUGGGAUCAUUGCCCUG (SEQ ID NO: 62).
[23] The vaccine according to [19] for preventing and/or treating a disease related to β-amyloid peptide, wherein the mRNA codes for a partial peptide inducing an antibody specifically binding to β-amyloid peptide, and having an amino acid sequence SGYEVHHQKLVFFA (SEQ ID NO: 50), or a mutant partial peptide having one or several substitutions, deletions, or additions of amino acid residues within the amino acid sequence of the partial peptide, and having an equivalent activity to the partial peptide.
[24] The vaccine according to [23], wherein the mRNA is UCAGGAUAUGAAGUUCAUCAUCAAAAAUUGGUGUUCUUUGCA (SEQ ID NO: 63).
[25] The vaccine according to [19] for preventing and/or treating a disease related to human PD-L1 protein, wherein the mRNA codes for a partial peptide inducing an antibody specifically binding to human PD-L1 protein, and having an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), or a mutant partial peptide having one or several substitutions, deletions, or additions of amino acid residues within the amino acid sequence of the partial peptide, and having an equivalent activity to the partial peptide.
[26] The vaccine according to [19] for preventing and/or treating a disease related to human PD-1 protein, wherein the mRNA codes for a partial peptide inducing an antibody specifically binding to human PD-1 protein, and having an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), or a mutant partial peptide having one or several substitutions, deletions, or additions of amino acid residues within the amino acid sequence of the partial peptide, and having an equivalent activity to the partial peptide.

### EFFECT OF INVENTION

According to the present invention, low molecular peptides that induce antibodies similar to therapeutic antibodies without chemically binding to carrier proteins such as KLH or CRM197, can be provided. The peptides of the present invention are useful for the development of peptide vaccines and RNA vaccine therapies for target proteins.

### BRIEF DESCRIPTION OF DRAWINGS

[FIG. 1] Figure 1 is a graph showing the reactivity of antisera A1 and A2 against each antigen peptide in group A administered with mixed peptides (10 types).
[FIG. 2] Figure 2 is a graph showing the reactivity of antisera B1 and B2 against each antigen peptide in Group B administered with mixed peptides (12 types).
[FIG. 3] Figure 3 is a graph showing the reactivity of antiserum C1 and C2 against each antigen peptide in group C administered with mixed peptides (12 types).
[FIG. 4] Figure 4 is a graph showing the reactivity of antisera D1 and D2 against each antigen peptide in group D administered with mixed peptides (12 types).
[FIG. 5] Figure 5 is a graph showing the reactivity of eight antisera A1 to D2 at a 100-fold dilution against human TNFα (hTNFα).
[FIG. 6] Figure 6 is a photograph demonstrating the reactivity of antisera A1 to D2 against human TNFα using Western blotting.
[FIG. 7] Figure 7 is a graph showing the results of the antibody titers against each human TNFα peptide measured in the antisera obtained by subcutaneously immunizing rabbits with mixtures E to H of human TNFα peptides. S/N ratio in the figure refers to the ratio of the antibody titer one week after the final administration to the antibody titer on the start date of administration.
[FIG. 8] Figure 8 is a graph showing the results of the antibody titers against human TNFα measured in antisera A to D obtained by subcutaneously immunizing rabbits with mixtures E to H of human TNFα peptides. S/N ratio in the figure refers to the ratio of the antibody titer one week after the final administration to the antibody titer on the start date of administration.
[FIG. 9] Figure 9 is a graph showing the results of the antibody titers against the human TNFα peptide measured in the antiserum obtained by subcutaneously immunizing rabbits with the human TNFα peptide alone. S/N ratio refers to the ratio of the antibody titer one week after the final administration to the antibody titer on the start date of administration.
[FIG. 10] Figure 10 is a graph showing the results of the antibody titers against human PD-1 (hPD-1 peptide) mixtures A2 to D2 measured in the antisera obtained by subcutaneously immunizing rabbits with hPD-1 peptide mixtures A2 to D2. S/N ratio refers to the ratio of the antibody titer one week after the final administration to the antibody titer on the start date of administration.
[FIG. 11] Figure 11 is a graph showing the results of the antibody titers against the mouse PD-1 (mPD-1 peptide) mixtures A3 to D3 measured in the antisera obtained by subcutaneously immunizing rabbits with the mPD-1 (mPD-1 peptide) mixtures A3 to D3. S/N ratio refers to the ratio of the antibody titer one week after the final administration to the antibody titer on the start date of administration.
[FIG. 12] Figure 12 shows the results of flow cytometry evaluation of the binding affinity of mouse PD-1 (mPD-1 peptide) mixtures A3 to D3 against EL-4 cells.
[FIG. 13] Figure 13 is a graph showing the tumor growth inhibition effect induced by the immunization with mouse PD-1 peptide mixtures A4 to D4.
[FIG. 14] Figure 14 is a graph showing the results of the antibody titers against hPD-L1 (hPD-L1 peptide) mixtures A4 to D4 measured in the antisera obtained by subcutaneously immunizing rabbits with hPD-L1 peptide mixtures A4 to D4. S/N ratio refers to the ratio of the antibody titer one week after the final administration to the antibody titer on the start date of administration. In the figure, "hPL8-22" refers to "hPD-L1(18-22)", and the same is applied to the others.
[FIG. 15] Figure 15 is a graph showing the anti-hTNFα antibody titers obtained by immunizing rabbits with hTNFα(143-157), hTNFα(138-157), hTNFα(133-157), or hTNFα(128-157).
[FIG. 16] Figure 16 is a graph showing the anti-hTNFα antibody titers obtained by immunizing rabbits with multiple hTNFα peptides of 30-amino acid in length (30AA).
[FIG. 17] Figure 17 is a graph showing the anti-human TNFα antibody titers against obtained by immunizing with inactivated human TNFα (V91D).
[FIG. 18] Figure 18 is a graph showing the neutralizing activity against human TNFα in the antisera obtained by immunizing with inactivated human TNFα (V91D).
[FIG. 19] Figure 19 is a graph showing the blood clearance effect on human TNFα in the antisera obtained by immunizing with human TNFα peptides in 30AA length.

### ASPECTS FOR CARRYING OUT INVENTION

### <Methods for preparing partial peptides having abilities to induce antibodies against target proteins>

The present invention aims at inducing antibodies against a target protein, specifically using peptides as antigens derived from the protein.

The present invention relates to a peptide and a peptide mixture composition for inducing antibodies against a target protein by administering a partial peptide within the target protein. Not all peptides prepared by the method for the preparation according to the present invention have any antigen-inducing ability, but rather, according to the present invention, it is assumed that only peptides or peptide mixtures having a high antibody-inducing ability without carrier proteins (carriers) are selected and administered to patients taking account of Quality Of Life (QOL).

In one aspect, the present invention relates to a method for preparing a partial peptide within a target protein, which induces an antibody specific to the target protein, which comprises:
1) providing a plurality of partial peptides of 5-30 amino acid residues within the amino acid sequence of the target protein, wherein each partial peptide may overlap with an adjacent different partial peptide by a specific number of amino acid residues at its N-terminus or C-terminus, and wherein the plurality of the partial peptides covers the entire region of the amino acid sequence of the target protein;
2) making a mixture containing the 4 to 12 partial peptides obtained;
3) immunizing non-human mammals by use of the obtained mixture;
4) determining whether the mixture induces any antibody specific to the target protein by the immunization; and
5) identifying the partial peptide that induces the specific antibody from the mixture that has been determined to induce the antibody.

According to the present invention, it is not necessary to chemically bond low molecular weight peptides to carrier proteins such as KLH or CRM197, allowing for providing peptides that can induce antibodies similar to therapeutic antibodies. The advantages of the present invention that does not utilize carrier proteins are as follows:
1. It is possible to prevent side effects caused by the administration of carrier proteins not originally required for the immune response, which are foreign proteins.
2. It is possible to prevent rapid increases in antibody levels and unexpected antibody excesses, which could occur due to the addition of carrier proteins. In general, the vaccines for prevention against infections are administered to reach the antibody excesses through 1 to 3 doses. On the other hand, the vaccines for therapy, which are antibody vaccines against substances necessary for the organism, such as physiological active substances, are administered to induce the desired antibody titer while monitoring the antibody titer. Since both vaccines for prevention against infections and vaccines for therapy require multiple doses, it is necessary to avoid rapid increases in antibody levels due to the carrier proteins.
3. It is possible to significantly reduce production costs, since the active ingredients as pharmaceutical active components can be chemically synthesized medicaments rather than biopharmaceuticals.

The followings describe the method for preparation according to the present invention by each step.

### Step 1)

In the present invention, the target proteins include, but not limited to, the proteins recognized by antibodies that are used in therapy and marketed as drugs. Alongside antibody drugs, the examples of the target proteins include, for example, EGF receptor proteins in trastuzumab (Herceptin, anti-EGF receptor), CD20 protein in rituximab (Rituxan/anti-CD20), TNFα protein in infliximab (Remicade/anti-TNFα), IL6 receptor protein in tocilizumab (Actemra/anti-IL6 receptor), VEGF protein in bevacizumab (Avastin/anti-VEGF), TNFα protein in adalimumab (Humira/anti-TNFα), IL-1β protein in canakinumab (Ilaris/anti-IL-1β), CCR4 protein in mogamulizumab (Poteligeo/anti-CCR4), PD-1 protein in nivolumab (Opdivo/anti-PD-1), and amyloid β protein in lecanemab (Aducanumab/amyloid β), among others.

The optimal number of the amino acid residues in the peptide segment of the amino acid sequence within the target protein ranges from a synthesizable number (for example, about 30 or more) to the number of amino acid residues required for an epitope, which is 3 to 7. In general, the number of amino acid residues in the functional peptides, specifically the "killer peptides" and "helper peptides", is approximately 9 and 15, respectively. When preparing peptide antibodies against a target protein, peptides consisting of around 10 to 20 amino acids are often used. Further, the synthesis of peptides with strong hydrophobicity due to not only the number of amino acid residues but also the amino acid sequence itself necessitates the crafting of solubilization. In conclusion, the number of amino acid residues in the peptide is considered to range from 3 to 30 or more. Peptides with a higher number of amino acid residues exhibit a stronger antigenicity and a higher antibody induction capability, but, the synthesis process of the same results in a greater number of by-products, leading to lower recovery rates and purities compared to peptides with about 10 residues. In the examples as described herein, the synthesis was attempted with a target of 9 amino acid residues for the killer peptides, and with a target of an average of 15 amino acid residues for the helper peptide and peptide antibody.

According to the present invention, each partial peptide may overlap with an adjacent different partial peptide by a specific number of amino acid residues at its N-terminus or C-terminus. It is important to select partial peptides in such a way that more than 50%, preferably more than 60%, and even more preferably more than 70% of the sequences of each partial peptide, overlap with each other; however, the overlap in the partial peptides does not need to be the same percentage.

According to the present invention, a plurality of partial peptides is prepared to cover the entire region of the amino acid sequence of the target protein. The preparation of partial peptides does not depend on the method, whether it is the synthesis described below or the degradation of proteins. It should be important to synthesize the partial peptides from the N-terminus without gaps. For example, when extracting initially a partial peptide as killer peptides and helper peptides, simultaneously a new partial peptide from the peptide sequences between those peptides are selected and synthesized.

To select partial peptides, the publicly available Immune Epitope Databases, such as the IEDB and SYFPEITHI can be utilized. For example, SYFPEITHI is used to insert the amino acid sequence of the target protein, to extract the partial peptides of killer peptides (9) and helper peptides (15), and further to select them to ensure that the amino acid sequences do not overlap. Without being particular about the software, it is acceptable to determine the peptides by dividing the number of amino acid residues of the target protein into approximately 15 segments. In the following example, amyloid β consisting of 42 amino acids was divided into three parts: peptide (1-14), peptide (15-28), and peptide (29-42), along with providing peptides (8-21) and (22-35) overlapping those peptides, thereby making a total of five partial peptides.

### Step 2)

According to the present invention, a mixture containing the 4 to 12 partial peptides obtained is made. A plurality of mixtures of several partial peptides is made. The combination of the partial peptides to be used is flexible, as long as all partial peptides obtained are utilized without omission in making the mixtures. For example, when there are the 30 partial peptides of the derived protein and they are administered as a mixture of three groups of 10 types each, the partial peptides of the derived protein are arranged in order from the N-terminus, and each peptide is numbered from 1 to 30. For example, when the first group is defined as a mixture of partial peptides numbered 3n+1, the second group as a mixture of partial peptides numbered 3n+2, and the third group as a mixture of partial peptides numbered 3n+3 wherein n is an integer from 0 to 9, then the ten partial peptides that cover all the derived protein are evenly distributed.

The higher the number of partial peptides contained in the mixture, the better in terms of antibody induction. However, when the number of partial peptides is increased, the amount of each partial peptide contained in the peptide mixture would decrease, which may lower the potential for inducing the peptide antibodies that can originally be induced. Therefore, it may also be possible to limit the number of peptides. In general, there are from 5 to 20, preferably from 7 to 15, more preferably from 8 to 12, and typically 10 of them.

The mixture in Step 2) is subject to certain limitations in its total amount, considering the administration to animals. In the case of general carrier proteins and peptides, 100 µg of the peptide is sufficient. However, the unmodified peptides according to the present invention would be unstable due to the serum protease and the excretion from the kidneys. When the number of partial peptides contained in the mixture is small, the amount of each partial peptide administered would be increased, which lead to inducing more likely the antibodies if the partial peptide administered is effective in inducing antibodies. On the other hand, when the number of partial peptides is increased, the amount of each partial peptide would be decreased, which may lead to a reduction in the rate of antibody induction. Considering these factors, the total amount of the mixture should be determined.

### Step 3)

In the step, the obtained mixture is used to immunize non-human mammals.

Examples of non-human mammals include a mouse, a rat, a rabbit, a dog, or a monkey. The rabbit or the monkey is preferable. When the target protein is of human origin, it is preferable to immunize the monkey and obtain antibodies that are specific to the target protein since the amino acid sequence of the target protein is almost identical to that of the homologous protein in monkeys.

An adjuvant is essential in immunization. The adjuvant is classified into the sedimentary adjuvant and the oily adjuvant. A representative example of the former is an aluminum compound, which is an inorganic suspension agent that adsorbs antigens and is used in many vaccines. A representative example of the latter is an incomplete Freund's adjuvant, which comprises paraffin and Arachis oil, and forms micelles by encapsulating the antigen solution, thereby creating an emulsified oil emulsion. However, this becomes a cloudy emulsion which would be a highly viscous fluid, and so it may cause pain during the inoculation. It also has the property of being difficult to disperse within the body and of tending to remain at the site of administration, where it may harden. The Freund's adjuvant is commonly used in animal immunology, but it is not widely used in humans. Only incomplete Freund's adjuvant Montanide is used in cancer peptide vaccine therapy, in which killer peptides are micellized in oil components to remain them at the injection site, and the encapsulation in the micelles prevent their degradation by serum proteases. Considering the dosage form of the peptide vaccine to be an oil-based adjuvant, it is expected to develop adjuvants that are equal to or superior to Montanide. In the Examples hereinafter, the peptide mixtures were administered to the animals using the simple incomplete Freund's adjuvant of components.

### Step 4)

Here, it is determined whether the aforementioned mixture induces any antibodies specific to the target protein by the corresponding immunization.

The determination of whether specific antibodies are induced, that is, the detection of peptide antibodies, can be performed using various immunoassays such as immunoassay against solid-phase peptides or target proteins (peptide ELISA), Western Blotting using nitrocellulose (WB), immunoprecipitation (IP), receptor assays (RRA), as well as neutralization activity measurement methods using cells (such as L929), CDC activity measurement methods, ADCC activity measurement methods, and evaluations in animal models regarding the reduction in blood concentration of actual solubilized target proteins or the promotion of clearance. In this regard, the induced peptide antibodies are utilized to bind to a membrane-type target protein on a target protein-producing cell, thereby reducing the target protein-producing cells due to antibody-dependent cellular cytotoxicity (ADCC) by CTL cells and complement-dependent cytotoxicity (CDC) by complements (cellular immunity). On the other hand, neutralizing activity (humoral immunity) is utilized to bind to a secreted target protein and inhibit the binding to the target protein receptor. Additionally, it is also possible to utilize the formation of immune complexes by binding to the secreted target protein and the metabolization of the target protein, thereby reducing the soluble target protein.

In addition, the detection of peptide antibodies can be confirmed by immunohistochemistry (IHC) using cells or tissues expressing the target protein, or by flow cytometry (FCM) of cells in suspension (Maleki, L.A.); Majidi, J.; Baradaran, B.; Abdolalizadeh, J.; Akbari, A.M., Production and characterization of murine monoclonal antibody against synthetic peptide of cd34., Hum. Antibodies 2013, 22, 1-8.).

### Step 5)

In the step, the partial peptide that induces the specific antibody from the mixture that has been determined to induce the antibody is identified. The identification of partial peptides is typically conducted by using a plate solid-phased with the derived protein and identifying the peptides that inhibit the binding of the derived protein in the antiserum obtained from the immunization in step 3.

According to another embodiment of the present invention, a peptide that induces the antibodies against the target protein may be identified and prepared through the following processes. That comprises the first process to identify the antiserum comprising peptide antibodies using a plate solid-phased with the peptide, the second process to identify the antiserum comprising peptide antibodies that react with the target protein, and the third process to identify the peptides that inhibit the peptide antibody reaction using a plate solid-phased with the target protein.

In the first process, it is necessary to detect antibodies with higher sensitivity, since peptides consisting of around 15 amino acid residues are generally recognized as haptens that do not possess antigenicity. In general, a 96-well plate is used to prepare a plate solid-phased with the peptide wherein multiple peptides to be administered are individually added and adsorbed onto each well of the plate. The serum with high antibody titer from the whole blood is added to each well, and the reactivity with each peptide in the well is observed. When considering with higher sensitivity, the dilution factor of the serum should be examined at approximately 100 times. At this stage, the peptide that induces the antibodies among the multiple peptides administered may be identified, and at the same time, the antiserum comprising the peptide antibodies is identified. The serum obtained from the partial blood sampling of the blood collected one week after the administration of the peptide mixture solution can also be used in place of the serum obtained from the complete blood sampling.

In the second process, it is examined whether the antiserum comprising the peptide antibodies induced in the first process binds to the target protein. Detection can be conducted using an ELISA that employs a plate solid-phased with the target protein, similar to peptides, and a Western blotting method (WB) to observe the reactivity with the polymer target protein.

In the third process, the target protein is solid-phased on a plate, and during the addition of the antiserum that reacts with the target protein, multiple peptides are added in excess individually to identify the peptides that inhibit the binding of the antiserum to the target protein. The peptide antibodies induced by this peptide recognize the target protein. This conclusion can be confirmed by administering the peptide alone to the animal to see if antibodies that respond to the target protein are induced.

Determination of the amino acid sequence of a protein is described. The attempt to analyze the amino acid sequence has been conducted for a long time, with the first report made by Dr. Frederick Sanger in the U.K. who was awarded the Nobel Prize in Chemistry in 1958. Utilizing the fact that the attachment of a dinitrophenyl group (DNFB) to the amino group at the N-terminus of a peptide results in yellow color development, he established a method for determining the amino acid sequence of proteins. He used this method to successfully identify the primary structure of insulin for the first time, ultimately confirming that the protein is composed of linked amino acids. This method is called the Sanger method. Because the Edman method using phenylisothiocyanate (PITC) can determine the N-terminal amino acids with higher sensitivity, by the early 1980s, the amino acid sequences from the N-terminus of trace amounts of proteins began to be determined using protein/nucleic acid sequencers. The corresponding nucleic acid probe is synthesized from the determined N-terminal amino acid sequence, allowing for the capture of the target protein's mRNA (cDNA) from the human mRNA library, and enabling the determination of the complete amino acid sequence of the target protein through the analysis of that cDNA. The method for determining the base sequence of the DNA was also established by Sanger, which is a method for determining the base sequence of DNA using dideoxynucleotides.

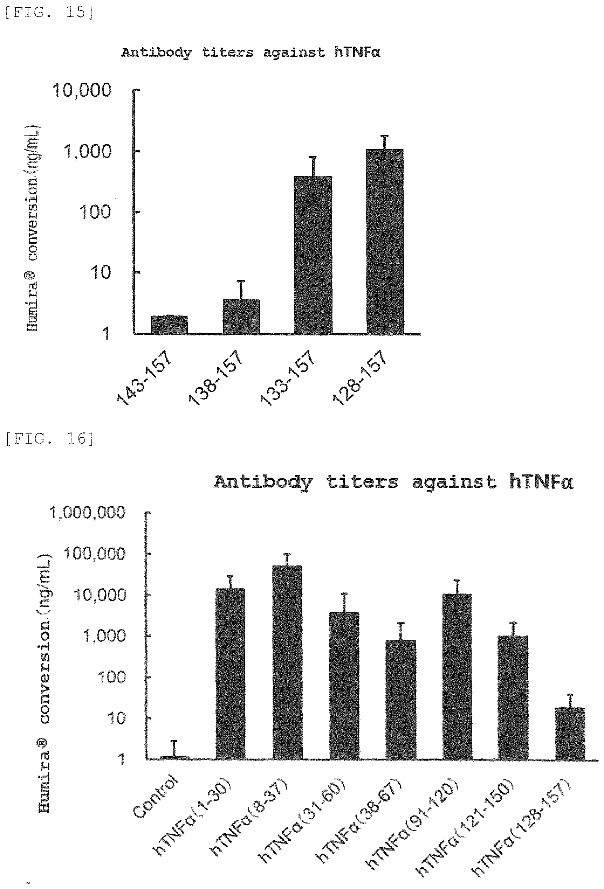

In another aspect, the present invention relates to the method according to the present invention which further comprises a step of adding an additional sequence to the identified partial peptide. In principle, when producing antibodies using carrier proteins, as the length of the peptide used for the antigen increased, the specificity of the antibodies tends to decrease. However, according to the present invention, in contrast to this, extending the peptide chain has been attempted in a method for enhancing the antibody-inducing activity of the peptide. In Example 14, the C-terminal peptide of TNFα with low induction of anti-TNF-α antibodies (TNF143-157) was selected as a peptide under consideration, and three TNF peptides TNF(138-157), TNF(133-157), TNF(123-157) were administered, each extended by five peptides at the N-terminal, to investigate the induction of antibodies. As a result, the peptide with the highest antibody titer against TNFα was found to be 30AA TNF(123-157) (Figure 15). The limit of amino acids for efficient peptide synthesis is approximately 30AA, and therefore 30AA peptides were efficiently selected and synthesized from the full-length TNFα once again, and comprehensively observed the antibody titers against TNFα, resulting in Figure 16. That is, according to the present invention, the term "additional sequence" refers to a sequence that provides a strongly specific antibody even when the length of the peptide used as the antigen is extended.

### <Partial peptides having abilities to induce antibodies against target proteins>

In another aspect, the present invention relates to a partial peptide within a target protein prepared by the method for preparation according to the present invention, and/or identified by the screening method according to the present invention described below, which induces an antibody specific to the target protein, or a mutant partial peptide having one or several substitutions, deletions, or additions of amino acid residues within the amino acid sequence of the partial peptide, and having an equivalent activity to the partial peptide. Specifically, the following peptides are provided.
A partial peptide that induces an antibody specifically binding to human TNFα, and has an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCQRETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), or A mutant partial peptide that has one or several substitutions, deletions, or additions of amino acid residues within the amino acid sequence of the partial peptide, and has an equivalent activity to the partial peptide;
A partial peptide that induces an antibody specifically binding to human β-amyloid peptide, and has the amino acid sequence SGYEVHHQKLVFFA (SEQ ID NO: 50), or A mutant partial peptide having one or several substitutions, deletions, or additions of amino acid residues in the amino acid sequence of the partial peptide, and has an equivalent activity to the partial peptide;
A partial peptide that induces an antibody specifically binding to human PD-L1, and has an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), or a mutant partial peptide that has one or several substitutions, deletions, or additions of amino acid residues in the amino acid sequence of the partial peptide, and has an equivalent activity to the partial peptide;
A partial peptide that induces an antibody specifically binding to human PD-1, and has an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), or a mutant partial peptide that has one or several substitutions, deletions, or additions of amino acid residues within the amino acid sequence of the partial peptide, and has an equivalent activity to the partial peptide; and
A partial peptide that induces an antibody specifically binding to mPD-1, and has an amino acid sequence selected from GWLLEVPNGPWRSLT (SEQ ID NO: 55), ATFTCSLSNWSEDLM (SEQ ID NO: 56), KQAAFCNGLSQPVQD (SEQ ID NO: 57), and FHMNILDTRRNDSGI (SEQ ID NO: 58), or a mutant partial peptide that has one or several substitutions, deletions, or additions of amino acid residues in the amino acid sequence of the partial peptide, and has an equivalent activity to the partial peptide.

The partial peptides within the target proteins that induce antibodies specific to the target proteins, which identified by the screening method of the present invention and/or the mutant partial peptides can be utilized as an antigen in vaccines with the same as an active ingredient.

The mutant partial peptides that have one or several substitutions, deletions, or additions of amino acid residues in the amino acid sequence of the partial peptides prepared by the preparation method of the present invention and/or identified by the screening method, and have an equivalent activity to the partial peptides, can be prepared by a method known to those skilled in the art using the partial peptides. For example, in the case of the substitution of amino acid residues, "conservative substitution" well-known to the those skilled in the art may be utilized. Specifically, the substitution comprises:
(1) identifying the partial peptide that is prepared by the preparation method and/or identified by the screening method according to the present invention;
(2) replacing the selected amino acid(s) within the amino acid sequence of the identified partial peptide with other amino acid(s) known to have similar properties to those selected amino acid(s); and
(3) testing whether the obtained mutant peptide has the desired activity.

### <Screening method for partial peptides having abilities to induce antibodies against target proteins>

In another aspect, the present invention relates to a screening method for a partial peptide within a target protein that induces antibodies specific to the target protein, which comprises:
1) providing a plurality of partial peptides of 5-30 amino acid residues within the amino acid sequence of the target protein, wherein each partial peptide may overlap with an adjacent different partial peptide by a specific number of amino acid residues at its N-terminus or C-terminus, and wherein the plurality of the partial peptides covers the entire region of the amino acid sequence of the target protein;
2) making a mixture containing the 4 to 12 partial peptides obtained;
3) immunizing non-human mammals by use of the obtained mixture;
4) determining whether the mixture induces any antibody specific to the target protein by the immunization; and
5) identifying the partial peptide that induces the specific antibody from the mixture that has been determined to induce the antibody.

In another embodiment, the present invention relates to the method according to the present invention, which further comprises;
6) a step of adding an additional sequence to the identified partial peptide.

The explanations of the other terms are the same as those in the method for the preparation according to the present invention.

### <Peptide vaccines>

In another embodiment, the present invention relates to a vaccine for preventing and/or treating a disease related to a target protein, which comprises a partial peptide within the target protein identified by the method for preparation according to [1], which induces an antibody specific to the target protein, or a mutant partial peptide. Specifically, the following vaccines are provided.
A vaccine according to the present invention for preventing and/or treating a disease related to human TNFα, wherein the partial peptide induces an antibody specifically binding to human TNFα, and has an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCQRETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), and wherein the mutant partial peptide has one or several substitutions, deletions, or additions of amino acid residues within the amino acid sequence of the partial peptide, and has an equivalent activity to the partial peptide;
A vaccine according to the present invention for preventing and/or treating a disease related to human β-amyloid peptide, wherein the partial peptide induces an antibody specifically binding to human β-amyloid peptide, and has an amino acid sequence SGYEVHHQKLVFFA (SEQ ID NO: 50), and wherein the mutant partial peptide has one or several substitutions, deletions, or additions of amino acid residues within the amino acid sequence of the partial peptide, and has an equivalent activity to the partial peptide;
A vaccine according to the present invention for preventing and/or treating a disease related to human PD-L1 protein, wherein the partial peptide induces an antibody specifically binding to human PD-L1 protein, and has an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), and wherein the mutant partial peptide has one or several substitutions, deletions, or additions of amino acid residues within the amino acid sequence of the partial peptide, and has an equivalent activity to the partial peptide; and
A vaccine according to the present invention for preventing and/or treating a disease related to human PD-1 protein, wherein the partial peptide induces an antibody specifically binding to human PD-1 protein, and has an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), and wherein the mutant partial peptide has one or several substitutions, deletions, or additions of amino acid residues within the amino acid sequence of the partial peptide, and has an equivalent activity to the partial peptide.

The vaccine according to the present invention can comprise a plurality of the partial peptides of the present invention. For example, in the treatment of rheumatoid arthritis, two classes of antibody drugs that inhibit the binding of TNFα and IL-6 receptors are applied, which simultaneous administration of the two drugs is expected to enhance the therapeutic effect. However, the antibody drugs are expensive, and so the treatment is often limited to one drug. On the other hand, the peptides of the present invention can induce separate antibodies, and therefore, for example, by creating a cocktail vaccine that includes multiple partial peptides, such as five partial peptides, it becomes possible to conduct vaccine therapy with a single formulation.

Synthesis of the peptide in the vaccine according to the present invention is described below.

According to the solid-phase synthesis of peptides, peptides composed of several to over 30 amino acids can be synthesized by sequentially coupling α-amino acids with protected side chains to an insoluble resin support using a linker. Specifically, after removing the amino acid protecting group at the N-terminus of the amino acid at the C-terminus of the peptide sequence, the amino acid is condensed using a coupling reagent and a pre-activated amino acid derivative from the second position at the C-terminus to elongate the peptide chain, and, by repeating the process of removing protecting groups and coupling, after the final amino acid is condensed, the resin is cleaved from the solid-phase surface (C-terminus), thereby providing the desired peptide. The protecting groups for the amino acids include Boc-group (to be removed by an acid) and Fmoc-group (to be removed by a base), and the amino acids by Boc-group, which create many by-products during acid treatment, has no longer been used in many cases. The peptide synthesis apparatus utilizing the amino acids by Fmoc-group has become widely used, makes peptide synthesis inexpensive, and also available for purchase. As a result, peptide antibodies can also be prepared easily and inexpensively. Further, through the synthesis of peptide oligomers, it has become possible to synthesize three-dimensional similar peptides (dendrimers) with enhanced antigenicity (Sebestik, J.; Niederhafner, P.; Jezek, J. Peptide and glycopeptide dendrimers and analogous dendrimeric structures and their biomedical applications. Amino Acids 2011, 40, 301-370). Furthermore, it has become possible to synthesize peptides using non-natural D-amino acids or isotopically labeled amino acids (13C, 15N, 2H), thereby expanding the research area for the pharmaceutical development of peptides themselves.

Peptide synthesis may be performed by utilizing widely the synthesis contract services. For example, it is possible to request the services of GenScript Corporation to obtain high-purity peptides through mass spectrometry analysis (the Example of the present invention). It is also possible to enhance the stability of the peptides in serum by converting them into D-amino acids, and acetylating/amidating them at the N/C terminus, and to increase in the antigenicity by polymerizing the peptides or adding an adjuvant peptide.

Since the synthesized and freeze-dried peptides are not dissolved in pure water in many cases, it is checked if basic peptides are dissolved in acetic acid (approximately 10% v/v) or TFA (0.1% v/v), and if acidic peptides are dissolved in ammonia water (1% NH4OH). Highly hydrophobic peptides or neutral peptides may be dissolved by gradually adding 5% to 50% of acetonitrile or by adding isopropanol, DMF, or DMSO followed by ultrasonic treatment. Further, peptides that form stable secondary structures are well dissolved by adding denaturants such as urea or guanidine. A single peptide, a mixture with a basic peptide, or a mixture with an acidic peptide may be dissolved in acetic acid or ammonia water, and a mixture of a basic peptide, an acidic peptide, and a neutral peptide is hardly to be dissolved in acetic acid or ammonia water. When a large number of peptides, that is, 10 or more peptides are solvolyzed, it is difficult to achieve solubilization with acetic acid or ammonia water; instead, isopropanol, DMF, or DMSO would be used. As an example, the freeze-dried peptide may be dissolved in DMSO at a concentration of 10 mg/mL and the solution may be stored at -30°C until use. When administering to animals, a peptide mixture for screening was prepared by dissolving 4 to 10 peptides in DMSO. Once the specific peptide is determined, the specific solubilization conditions for the same may be found. When the sequence contains the oxidation-sensitive amino acids; cysteine (C), methionine (M), and tryptophan (W), a deoxygenated solvent (to be degasified by reduced pressure, or bubbled with nitrogen, helium or argon) may be used to maintain the stability of the peptide, allowing for long-term use.

The vaccine of the present invention can significantly induce the production of antibodies against the target protein, when administered to mammals in conjunction with a carrier protein (or a protein containing T cell epitopes). According to a preferred embodiment of the present invention, the carrier protein is selected from the group consisting of Keyhole Limpet Hemocyanin (KLH), Tetanus Toxoid (TT), Diphtheria Toxoid (DT), or any other protein or peptide containing T cell epitopes.

According to a preferred embodiment of the present invention, the enhancement of the antigenicity of the peptide can be achieved by adding a carrier protein and mixing an adjuvant.

Generally, peptides themselves lack sufficient complexity, and therefore they are not considered to induce an immune response nor elicit specific antibodies. Therefore, when producing peptide antibodies in animals, it is necessary to chemically conjugate the peptide to a carrier protein. Examples of the carrier include keyhole limpet hemocyanin (KLH), bovine serum albumin (BSA), ovalbumin (OVA), and detoxified diphtheria protein (CRM197). The CRM197 is used as a carrier protein for the pneumonia vaccine. The crosslinking method of peptides and carrier proteins is mainly performed using the reagents such as EDC (N-Ethyl-N'-3-dimethylaminopropyl carbodiimide), sulfo-SMCC (sulfosuccinimidyl-4-(N-maleimidomethyl)cyclohexane-1-carboxylate, and glutaraldehyde. For reference, the EDC crosslinking agent reacts with the effective carboxyl group on the carrier protein or the peptide hapten to form an active O-acylisourea intermediate, and then it reacts with a primary amine to form an amide bond and a soluble urea byproduct. The peptide-carrier protein immunogen can achieve a higher degree of polymerization by extending the reaction time, meaning that the degree of polymerization can be adjusted by the crosslinking time. The sulfo SMCC crosslinking agent is a heterobifunctional crosslinker that contains a maleimide group that reacts with thiol groups and a succinimidyl (NHS) ester that reacts with amino groups. After initially reacting the crosslinking agent with the carrier protein, which has many amines, the agent is reacted with peptides containing reducing terminal cysteines, allowing all peptide molecules to be conjugated with a uniform orientation as intended. The glutaraldehyde crosslinking agent can crosslink peptides and carrier proteins through both amines, and randomly crosslink the N-terminal of the peptide and the lysine residues, and the lysine group on the surface of the carrier protein. The disuccinimidyl suberate (DSS) reagent also performs similar cross-linking.

According to a preferred embodiment of the present invention, the peptide in the vaccine of the present invention is formulated with an adjuvant, and preferably, it is formulated by being adsorbed to aluminum sulfate.

As to the dosage of the peptide in the vaccine of the present invention, the optimal dosage is expected to be in the range of several micrograms to several milligrams, in view of the fact that specific antibodies are empirically observed in rabbits at several micrograms per body weight, when the peptide is cross-linked to the carrier protein, and that the maximum dosage in clinical trials of cancer peptide vaccines is 3 mg per body weight.

In a preferred embodiment, the vaccine of the present invention comprises the peptide of the present invention in an amount ranging from 1 µg to 1000 mg, preferably from 10 µg to 100 mg, particularly from 100 µg to 10 mg, or alternatively, for example, from 1 nmol to 1 mmol, preferably from 10 nmol to 100 µmol, and particularly from 100 nmol to 10 µmol. The peptide of the present invention is, per dose, preferably between 100 µg and 10 mg, more preferably between 300 µg and 3 mg. Typically, the vaccine may also comprise adjuvants, such as buffers, stabilizers, etc.

Administration interval of the vaccine according to the present invention needs to be short in order to raise the antibody titer quickly, for providing a preventive effect. The mixed vaccines wherein diphtheria, whooping cough, tetanus, poliovirus and the like are inactivated, which have high antigenicity, are administered subcutaneously three times at intervals of 3 to 8 weeks starting from 6 months of age, with a fourth dose given approximately one year after the third. The genetically engineered proteins (HBs antigen), which have lower antigenicity than the mixed vaccines, are administered via intramuscular injection for the second dose four weeks later and for the third dose six months later. The peptide vaccine is for treatment, and so the peptide is administered subcutaneously every two weeks in a formulation with low antigenicity. Antibody titers are monitored starting one month after administration, and when a certain level of the titer is reached, the administration is stopped, and the patient is placed under observation. When the antibody titer decreases, administration would be initiated again.

Examples of the method for administering the peptide in the vaccine of the present invention include intradermal administration (to the very superficial layer of the skin), subcutaneous administration (to the fat tissue just beneath the skin), intramuscular administration (to the muscle layer just beneath the fat tissue), intravenous administration (inserted directly into the vein) by injections, as well as patch administration (transdermal absorption) and sustainedrelease formulations. In the clinical trials of cancer peptide vaccines, intradermal administration, which is expected to provide immune effects, was performed, but, due to the residual fatty substances at the injection site leading to a decrease in the patients' Quality Of Life (QOL) and the complexity of administration, it has been shifting towards subcutaneous administration.

Liposome-like preparations in the vaccine of the present invention can be prepared by encapsulating the peptide of the present invention in a liposome. In this regard, liposome is a spherical vesicle that has at least one lipid bilayer, contains an aqueous phase inside, and can carry peptides on its surface, in the lipid phase, in the aqueous phase, or in all of them. The method for encapsulating peptides may comprise adding the peptides during the hydration of the lipid film to prepare the liposomes, or adding the peptides after the preparation of the liposomes. Peptides that are not encapsulated in the liposome can be removed by dialysis, molecular sieve chromatography, or ultracentrifugation.

The liposome-like preparations of the present invention can be administered to humans by conventional immunization methods. The conventional immunization methods refer to the intradermal administration (the very superficial layer of the skin), subcutaneous administration (the fatty tissue just beneath the skin), intramuscular administration (the muscle layer just below the fatty tissue), intravenous administration (directly inserted into the vein), as well as nasal administration, pulmonary administration, oral administration, and patch administration (transdermal absorption).

### <RNA vaccines>

According another embodiment of the present invention, the mRNA encoding the peptide of the present invention can be used as an active ingredient of the vaccine. mRNA that expresses peptides is directly injected into the living body to induce the expression of peptides, thereby eliciting antibodies against peptides that have no biological activity or against proteins that have only very weak biological activity. In this case, lipid nanoparticles (LNP) or liposomes are used to perform the administration, similarly to typical mRNA vaccines.

The LNP is a nanoparticle with a diameter ranging from 10 nm to approximately 1000 nm, primarily composed of lipids. As a non-viral drug delivery system (DDS), it is utilized for the delivery of nucleic acid drugs. Nucleic acids such as mRNA are easily degraded in the living body, and delivery technology is essential for their pharmaceutical application. LNPs composed of four types of lipids are commonly used as delivery vehicles for mRNA. The four types of lipids are "ionizable lipids" (pH-sensitive lipids) that protect nucleic acids and promote escape from endosomes, "phospholipids" and "cholesterol" that stabilize LNPs, and "PEGylated lipids" that suppress interactions with proteins in plasma and extend the half-life in the bloodstream (https://bio.nikkeibp.co.jp/atcl/report/16/011900001/17/01/ 12/00080/) .

The roles of the four types of lipids are as follows.

The ionizable lipids are an important component of the LNP (35 to 50%), and serve two main roles. They are to bind the RNA and to enable the release of the RNA within cells. The pKa of lipids is an important factor because the LNPs should not exhibit toxicity, should be uncharged at neutral pH, and should be positively charged at low pH. Hundreds of lipids have been synthesized and screened by many research groups, and lipids with desirable properties and effects have been identified. Successful examples include ALC-0315, cKK-E12, SM-102, and Dlin-MC3-DMA.

The PEGylated lipids are incorporated to extend the circulation half-life in the body in a small amount as a PEG-derivatized lipids (0.5 to 3%). PEGylated lipids have been used for many years in the liposome drug delivery systems that create the so-called "stealth" liposomes. Further, the percentage of the PEGylated lipid affects the size of the LNP. Examples include ALC-0159, mPEG-DSPE, and

### mPEG-DMG.

Cholesterol is a structural "helper" lipid that constitutes an important part (40 to 50%) of the LNP, and it likely improves efficacy by promoting membrane fusion and facilitating endosomal escape.

Neutral phospholipids include synthetic phospholipids such as DSPC, DPPC, and DOPE (up to 10%), and are commonly used as "helper" lipids in the structure of LNP formulations, which promote cell binding.

To prepare the LNP comprising the mRNA, the lipids in the ethanol are rapidly mixed with the mRNA in the low pH buffer using a microfluidic mixer. The ionizable lipid is protonated and begins to encapsulate by binding to the mRNA. The pH is gradually raised to 7.4, and by removing the ethanol, the LNP is allowed for the formation. The efficiency of mRNA encapsulation is significantly higher when using microfluidic mixing compared to the conventional extrusion used for liposomes. The characteristics of the LNP formulation, such as particle size, surface charge, and tissue targeting, can be adjusted by the types and ratios of the lipids within the LNP.

One embodiment of the present invention relates to a vaccine for preventing and/or treating a disease related to a target protein, which comprises mRNA coding for a partial peptide within the target protein identified by the method for preparation according to claim 1, which induces an antibody specific to the target protein, or a mutant partial peptide, and preferably to a vaccine which comprises a plurality of the mRNAs coding for the partial peptides and/or the mutant partial peptides.

Specifically, the mRNA is the mRNA codes for a partial peptide inducing an antibody specifically binding to human TNFα, and having an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSATKEPCORETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), or a mutant partial peptide having one or several substitutions, deletions, or additions of amino acid residues within the amino acid sequence of the partial peptide, and having an equivalent activity to the partial peptide. More specifically, the mRNA is selected from GGGCAGCUCCAGUGGCUGAACCGCCGGGCCAAUGCCCUCCUGGCC (SEQ ID NO: 59), CCCUGCCAGAGGGAGACCCCAGAGGGGGCUGAGGCCAAGCCCUGG (SEQ ID NO: 60), CUCCAGUGGCUGAACCGCCGGGCCAAUGCCCUCCUGGCCAAUGGC (SEQ ID NO: 61), and GACUUUGCCGAGUCUGGGCAGGUCUACUUUGGGAUCAUUGCCCUG (SEQ ID NO: 62).

In a further embodiment, the mRNA codes for a partial peptide inducing an antibody specifically binding to β-amyloid peptide, and having an amino acid sequence SGYEVHHQKLVFFA (SEQ ID NO: 50), or a mutant partial peptide having one or several substitutions, deletions, or additions of amino acid residues within the amino acid sequence of the partial peptide, and having an equivalent activity to the partial peptide. More specifically, the mRNA is UCAGGAUAUGAAGUUCAUCAUCAAAAAUUGGUGUUCUUUGCA (SEQ ID NO: 63).

In a further embodiment of the present invention, it is possible to utilize a partial peptide of a mutant protein wherein the activity of a target protein which is recognized by the antibody drug is reduced by modifying several amino acid residues in the target protein, regardless the active component of the vaccine is a peptide or an mRNA. For example, in the case of human TNF-α, it is impossible to use human TNF-α as an antigen in its form, since when human TNF-α is administered to humans as it is, it would cause harm to humans due to its biological activity before inducing antibodies. On the one hand, the mutant protein wherein the activity of human TNF-α is reduced does not produce the biological activity of human TNF-α itself, and therefore, the mutant protein as it is or the mutant protein produced via mRNA can be used to obtain antibodies against the mutant protein. The mutant protein can be prepared through genetic engineering.

As a mutant protein, in the case of TNF-α, the following can be described:
TNF-α V91A:
   Amino acid sequence: 
VRSSSRTPSD KPVAHVVANP QAEGQLQWLN RRANALLANG VELRDNQLVV

PSEGLYLIYS QVLFKGQGCP STHVLLTHTI SRIAVSYQTK ANLLSAIKSP

CQRETPEGAE AKPWYEPIYL GGVFQLEKGD RLSAEINRPD YLDFAESGQV

YFGIIAL (SEQ ID NO: 64)
   Base sequence: 
GTCAGATCAT CTTCTCGAAC CCCGAGTGAC AAGCCTGTAG CCCATGTTGT
AGCAAACCCT CAAGCTGAGG GGCAGCTCCA GTGGCTGAAC CGCCGGGCCA
ATGCCCTCCT GGCCAATGGC GTGGAGCTGA GAGATAACCA GCTGGTGGTG
CCATCAGAGG GCCTGTACCT CATCTACTCC CAGGTCCTCT TCAAGGGCCA
AGGCTGCCCC TCCACCCATG TGCTCCTCAC CCACACCATC AGCCGCATCG
CCGTCTCCTA CCAGACCAAG GACAACCTCC TCTCTGCCAT CAAGAGCCCC
TGCCAGAGGG AGACCCCAGA GGGGGCTGAG GCCAAGCCCT GGTATGAGCC
CATCTATCTG GGAGGGGTCT TCCAGCTGGA GAAGGGTGAC CGACTCAGCG
CTGAGATCAA TCGGCCCGAC TATCTCGACT TTGCCGAGTC TGGGCAGGTC
 TACTTTGGGA TCATTGCCCT G (SEQ ID NO: 65).
TNF-α C69A C101A:
   Amino acid sequence: 
VRSSSRTPSD KPVAHVVANP QAEGQLQWLN RRANALLANG VELRDNQLVV

PSEGLYLIYS QVLFKGQGAP STHVLLTHTI SRIAVSYQTK VNLLSAIKSP

AQRETPEGAE AKPWYEPIYL GGVFQLEKGD RLSAEINRPD YLDFAESGQV

YFGIIAL (SEQ ID NO: 66)
   Base sequence:

GTCAGATCAT CTTCTCGAAC CCCGAGTGAC AAGCCTGTAG CCCATGTTGT
AGCAAACCCT CAAGCTGAGG GGCAGCTCCA GTGGCTGAAC CGCCGGGCCA
ATGCCCTCCT GGCCAATGGC GTGGAGCTGA GAGATAACCA GCTGGTGGTG
CCATCAGAGG GCCTGTACCT CATCTACTCC CAGGTCCTCT TCAAGGGCCA
AGGCGCCCCC TCCACCCATG TGCTCCTCAC CCACACCATC AGCCGCATCG
CCGTCTCCTA CCAGACCAAG GTCAACCTCC TCTCTGCCAT CAAGAGCCCC
GCCCAGAGGG AGACCCCAGA GGGGGCTGAG GCCAAGCCCT GGTATGAGCC
CATCTATCTG GGAGGGGTCT TCCAGCTGGA GAAGGGTGAC CGACTCAGCG
CTGAGATCAA TCGGCCCGAC TATCTCGACT TTGCCGAGTC TGGGCAGGTC
TACTTTGGGA TCATTGCCCT G (SEQ ID NO: 67).

In this aspect, examples of diseases related to target proteins according to the present invention include diseases related to human TNFα, diseases related to human β-amyloid peptide, diseases related to human PD-L1 protein, and diseases related to human PD-1 protein. Examples of the diseases related to human TNFα include rheumatoid arthritis, psoriasis, ankylosing spondylitis, intestinal Behçet's disease and the like, which are the applicable diseases for adalimumab (Humira/anti-TNFα) targeting TNFα; examples of the diseases related to human β-amyloid peptide include Alzheimer's type cognitive impairment, mild dementia and the like, which are the applicable diseases for lecanemab (Aducanumab/amyloid β) targeting human amyloid β peptide; and examples of the diseases related to human PD-L1 protein and/or human PD-1 protein include various cancers, such as malignant melanoma, specifically unresectable malignant melanoma and unresectable advanced or recurrent non-small cell lung cancer, which are the applicable diseases for nivolumab (Opdivo/anti-PD-1) targeting PD-1.

In additions, examples of diseases related to the target protein according to the present invention include, but not limited to, breast cancer, which is an applicable disease for trastuzumab (Herceptin, anti-EGF receptor), CD20-positive B-cell non-Hodgkin lymphoma, which is an applicable disease for rituximab (Rituxan/anti-CD20), rheumatoid arthritis, which is an applicable disease for infliximab (Remicade/anti-TNFα) and tocilizumab (Actemra/anti-IL6 receptor), recurrent colorectal cancer, which is an applicable disease for bevacizumab (Avastin/anti-VEGF), cryopyrin-associated periodic syndrome, which is an applicable disease for canakinumab (Ilaris/anti-IL-1β), and CCR4-positive adult T-cell leukemia/lymphoma, which is an applicable disease for mogamulizumab (Poteligeo/anti-CCR4), among others.

The antibodies obtained from the partial peptides or mutant partial peptides of the present invention, while not being bound by any special theory, demonstrate neutralizing activity by binding to the target protein. On the other hand, it is believed that the antibodies are metabolized by macrophages in the tissues and in the liver, as they form immune complexes with the target proteins. The antibodies obtained by the present invention are expected to contribute to the disappearance of the target protein through ADCC or CDC, even if the sites recognized by the antibodies are not the binding sequence on the target protein recognized by the receptor (Example 17). The clearance (metabolic) activity is considered to be a neutralization activity in a broad sense. That is, the antisera obtained by the present invention are expected to exhibit efficacy as pharmaceuticals through the clearance of the target protein.

As used herein, "treatment" means a method or process aiming at (1) delaying or preventing the onset of diseases; (2) slowing or stopping the progression, aggravation or exacerbation of the symptoms of diseases; (3) inducing remission of the symptoms of diseases; or (4) facilitating the cure of diseases. Treatment may be given as a prophylactic measure before the onset of the disease or the condition, or treatment may be given after the onset of the disease.

According to the present invention, "prevention" means a prophylactic action for the development of diseases related to target proteins.

In another aspect, the present invention relates to a method for preventing and/or treating diseases related to target proteins, which comprises administering a vaccine comprising a partial peptide within the target protein or a mutant peptide that induces antibodies specific to the target protein, which is identified according to the method for the preparation of the present invention, to subjects in need of such treatment, preferably comprising administering an effective amount of the same to such subjects.

In further another aspect, the present invention relates to use of a vaccine comprising a partial peptide within the target protein or a mutant partial peptide that induces antibodies specific to the target protein, which is identified according to the method for the preparation of the present invention, for the manufacture of a medicament for the prevention and/or treatment of diseased related to target proteins.

"Pharmaceutically acceptable carrier or excipient" refers to a carrier medium that does not interfere with the biological activity of the active ingredient and does not exhibit excessive toxicity to the host at the concentration at which it is administered. This term includes solvents, dispersing agents, coating agents, antibacterial agents and antifungal agents, isotonic agents, adsorption delay agents, and the like. The use of such media and drugs for pharmaceutical active substances is well known in the art.

The correspondence between the three-letter and oneletter notations of the amino acids as used herein is as follows.
Alanine: Ala: A
Arginine: Arg: R
Asparagine: Asn: N
Aspartic Acid: Asp: D
Cysteine: Cys: C
Glutamine: Gln: Q
Glutamic Acid: Glu: E
Glycine: Gly: G
Histidine: His: H
Isoleucine: Ile: I
Leucine: Leu: L
Lysine: Lys: K
Methionine: Met: M
Phenylalanine: Phe: F
Proline: Pro: P
Serine: Ser: S
Threonine: Thr: T
Tryptophan: Trp: W
Tyrosine: Tyr: Y
Valine: Val: V

Hereinafter, the present invention will be described in more detail by way of the working examples, but it should be noted that these would not limit the scope of the present invention and merely illustrate the invention.

### Examples

In the following reference examples and wording examples, the reagents and the like were obtained from the manufacturers listed below, but the present invention is not limited to the use of them.
Peptides: GenScript, GenZyme
Complete/Incomplete Freund's Adjuvant: Fujifilm Wako Pure Chemical Corporation
MONTANIDE (Trademark) ISA 51 VG : SEPPIC

### Example 1

### Measurement of antibody titers of antisera obtained by subcutaneously immunizing rabbits with mixtures of hTNFα peptides

### Example 1-1: Synthesis of various hTNFα peptides

As human TNFα (hTNFα) peptides, a total of 46 peptides were synthesized, including the following: Synthesis 1 (10 types, Table 1), Synthesis 2 (11 types, Table 2), and Synthesis 3 (25 types, Table 3).

### Synthesis 1 (10 types)

The SYFPEITHI algorithm was used to identify HLA class II peptide sequences with high predicted affinity for the HLA-DRB1 molecule, and HLA class I peptide sequences with high predicted affinity for the HLA-A*2402 molecule, thereby determining the killer peptides (9 amino acid residues) and helper peptides (16 amino acid residues) within human soluble TNFα, respectively.
Human soluble TNFα (1-157): UniProt LinkDB: P01375





 (SEQ ID NO: 54)

Helper peptides and killer peptides that have homology between humans and mice were selected (Table 1).

**Table 1: Synthetic hTNFα Peptides: Helper Peptide (H) and Killer Peptide (K)**

| [Table 1] | | | |
|---|---|---|---|
| No | Peptide Name | Number of Amino acids | Peptides (Sites) |
| 1 | H(54-69) | 16 | GLYLIYSQVLFKGQG (54-69) (SEQ ID NO: 1) |
| 2 | H(65-80) | 16 | KGQGCPSTHVLLTHT (65-80) (SEQ ID NO: 2) |
| 3 | H(115-130) | 16 | YEPIYLGGVFQLEKG (115-130) (SEQ ID NO: 3) |
| 4 | H(120-135) | 16 | LGGVFQLEKGDRLSA (120-135) (SEQ ID NO: 4) |
| 5 | H(139-154) | 16 | PDYLDFAESGQVYFG (139-154) (SEQ ID NO: 5) |
| 6 | K(86-94) | 9 | SYQTKVNLL (86-94) (SEQ ID NO: 6) |
| 7 | K(112-130) | 9 | KPWYEPIYL (112-130) (SEQ ID NO: 7) |
| 8 | K(118-126) | 9 | IYLGGVFQL (118-126) (SEQ ID NO: 8) |
| 9 | K(124-132) | 9 | FQLEKGDRL (124-132) (SEQ ID NO: 9) |
| 10 | K(149-157) | 9 | QVYFGIIAL (149-157) (SEQ ID NO: 10) |

### Synthesis 2 (11 peptides)

Since the killer peptides and helper peptides synthesized in Synthesis 1 were unevenly located towards the C-terminal side of human soluble TNFα (1-157) (hereinafter, it may be referred to simply as TNFα), 11 additional peptides consisting of 15 amino acid residues distinct from the peptides of Synthesis 1 were selected, which are located from the N-terminal up to the C-terminal to cover the entire region of TNFα, with some overlapping sequences (Table 2).

**Table 2: 11 Covering TNF Peptides having 15 amino acid residues**

| [Table 2] | | | |
|---|---|---|---|
| No | Peptide Name | Number of Amino acids | Peptides (Sites) |
| 11 | T(1-15) | 15 | VRSSSRTPSDKPVAH (1-15) (SEQ ID NO: 11) |
| 12 | T(9-23) | 15 | SDKPVAHVVANPQAE (9-23) (SEQ ID NO: 12) |
| 13 | T(16-30) | 15 | VVANPQAEGQLQWLN (16-30) (SEQ ID NO: 13) |
| 14 | T(24-38) | 15 | GQLQWLNRRANALLA (24-38) (SEQ ID NO: 14) |
| 15 | T(31-45) | 15 | RRANALLANGVELRD (31-45) (SEQ ID NO: 15) |
| 16 | T(39-53) | 15 | NGVELRDNQLVVPSE (39-53) (SEQ ID NO: 16) |
| 17 | T(46-60) | 15 | NQLVVPSEGLYLIYS (46-60) (SEQ ID NO: 17) |
| 18 | T(76-90) | 15 | LTHTISRIAVSYQTK (76-90) (SEQ ID NO: 18) |
| 19 | T(91-105) | 15 | VNLLSAIKSPCQRET (91-105) (SEQ ID NO: 19) |
| 20 | T(100-114) | 15 | PCQRETPEGAEAKPW (100-114) (SEQ ID NO: 20) |
| 21 | T(130-144) | 15 | DRLSAEINRPDYLDF (130-144) (SEQ ID NO: 21) |

### Synthesis 3 (25 peptides)

Among the helper peptides (15 amino acid residues) that can be searched in SYFPEITHI, 25 peptides were selected, which were distinct from the peptides of Synthesis 1 and 2 (Table 3).

Specifically, among the human soluble TNFα (1-157), the first selection was made of 30 helper peptides in order of high scores in SYFPEITHI. In the search of Synthesis 1, the peptides with high-scoring scores around 50 were selected, and there were three peptides that matched between the 5 helper peptides in Synthesis 1 and the 30 helper peptides in Synthesis 3 (T(39-53), T(46-60), T(91-105)). On the other hand, there were two peptides that matched between the 11 peptides 15AA of Synthesis 2 and the 30 helper peptides of Synthesis 3 (H(54-69) and H(65-80)). Table 3 shows 25 peptides, excluding 5 types that matched with Synthesis 1 (3 types) and Synthesis 2 (2 types) from a total of the 30 peptides.

**Table 3: 25 TNFα helper peptides (15 amino acid residues)**

| [Table 3] | | | |
|---|---|---|---|
| No | Peptide Name | Number of Amino acids | Peptides (Sites) |
| 22 | H(10-24) | 15 | DKPVAHVVANPQAEG (10-24) (SEQ ID NO: 22) |
| 23 | H(13-27) | 15 | VAHVVANPQAEGQLQ (13-27) (SEQ ID NO: 23) |
| 24 | H(14-28) | 15 | AHVVANPQAEGQLQW (14-28) (SEQ ID NO: 24) |
| 25 | H(23-37) | 15 | EGQLQWLNRRANALL (23-37) (SEQ ID NO: 25) |
| 26 | H(26-40) | 15 | LQWLNRRANALLANG (26-40) (SEQ ID NO: 26) |
| 27 | H(32-46) | 15 | RANALLANGVELRDN (32-46) (SEQ ID NO: 27) |
| 28 | H(38-52) | 15 | ANGVELRDNQLVVPS (38-52) (SEQ ID NO: 28) |
| 29 | H(47-61) | 15 | QLVVPSEGLYLIYSQ (47-61) (SEQ ID NO: 29) |
| 30 | H(52-66) | 15 | SEGLYLIYSQVLFKG (52-66) (SEQ ID NO: 30) |
| 31 | H(53-67) | 15 | EGLYLIYSQVLFKGQ (53-67) (SEQ ID NO: 31) |
| 32 | H(60-74) | 15 | SQVLFKGQGCPSTHV (60-74) (SEQ ID NO: 32) |
| 33 | H(61-75) | 15 | QVLFKGQGCPSTHVL (61-75) (SEQ ID NO: 33) |
| 34 | H(71-85) | 15 | STHVLLTHTISRIAV (71-85) (SEQ ID NO: 34) |
| 35 | H(73-87) | 15 | HVLLTHTISRIAVSY (73-87) (SEQ ID NO: 35) |
| 36 | H(77-91) | 15 | THTISRIAVSYQTKV (77-91) (SEQ ID NO: 36) |
| 37 | H(80-94) | 15 | ISRIAVSYQTKVNLL (80-94) (SEQ ID NO: 37) |
| 38 | H(82-96) | 15 | RIAVSYQTKVNLLSA (82-96) (SEQ ID NO: 38) |
| 39 | H(88-102) | 15 | QTKVNLLSAIKSPCQ (88-102) (SEQ ID NO: 39) |
| 40 | H(90-104) | 15 | KVNLLSAIKSPCQRE (90-104) (SEQ ID NO: 40) |
| 41 | H(94-108) | 15 | LSAIKSPCQRETPEG (94-108) (SEQ ID NO: 41) |
| 42 | H(117-131) | 15 | PIYLGGVFQLEKGDR (117-131) (SEQ ID NO: 42) |
| 43 | H(120-134) | 15 | LGGVFQLEKGDRLSA (120-134) (SEQ ID NO: 43) |
| 44 | H(123-137) | 15 | VFQLEKGDRLSAEIN (123-137) (SEQ ID NO: 44) |
| 45 | H(129-143) | 15 | GDRLSAEINRPDYLD (129-143 (SEQ ID NO: 45) |
| 46 | H(141-155) | 15 | YLDFAESGQVYFGII (141-155) (SEQ ID NO: 46) |

### Example 1-2: Preparation of Various Peptide Mixtures

The 46 synthetic hTNFα peptides obtained from Synthesis 1, Synthesis 2 and Synthesis 3 were each weighed to approximately 1 mg and the weighed samples were placed into Eppendorf tubes, followed by being dissolved in dimethyl sulfoxide (DMSO) to a concentration of 1 mg/mL.

The 46 peptides were divided into four groups: Mixed Peptides (10) Dose Group A (10 peptides from Synthesis 1); Mixed Peptides (12) Dose Group B (11 peptides from Synthesis 2 and 1 peptide from Synthesis 3: wherein 1 peptide from Synthesis 3 H(117-131) does not overlap in sequence with the 11 peptides from Synthesis 2); and Mixed Peptides (12) Dose Group C and Mixed Peptides (12) Dose Group D (Dose Groups C and D were divided to evenly distribute the remaining 24 peptides from Synthesis 3: wherein the 25 peptides from H(10-24) to H(141-155) from Synthesis 3 were arranged in order, and the 24 peptides other than H(117-131) that do not overlap with the peptide sequences from Dose Group B were alternately assigned to Groups C and D) (Table 4), thereby preparing each peptide mixture. Specifically, for example, the peptide mixture of Mixed Peptides (10) Dose Group A was prepared by adding 0.1 mL of each DMSO solution of the 10 peptides to a new Eppendorf tube and mixing them. The obtained mixture was aliquoted into 10 Eppendorf tubes, with 0.1 mL each, 1/10th volume required for one immunization, and stored frozen to avoid freeze-thaw cycles of the peptide mixture.

**Table 4: The peptide mixture**

| [Table 4] | | | | |
|---|---|---|---|---|
| Number of peptides | Mixed Peptides (10) Dose Group A | Mixed Peptides (12) Dose Group B | Mixed Peptides (12) Dose Group C | Mixed Peptides (12) Dose Group D |
| | Peptides of Table 1 | Peptides of Table 2 + 1 | Half of Pept ides of Table 3 | Half of Peptides of Table 3 |
| 1 | H(54-69) | T(1-15) | H(10-24) | H(13-27) |
| 2 | H(65-80) | T(9-23) | H(14-28) | H(23-37) |
| 3 | H(115-130) | T(16-30) | H(26-40) | H(32-46) |
| 4 | H(120-135) | T(24-38) | H(38-52) | H(47-61) |
| 5 | H(139-154) | T(31-45) | H(52-66) | H(53-67) |
| 6 | K(86-94) | T(39-53) | H(61-75) | H(60-74) |
| 7 | K(112-130) | T(46-60) | H(73-87) | H(71-85) |
| 8 | K(118-126) | T(76-90) | H(80-94) | H(77-91) |
| 9 | K(124-132) | T(91-105) | H(88-102) | H(82-96) |
| 10 | K(149-157) | T(100-114) | H(94-108) | H(90-104) |
| 11 | | T(130-144) | H(120-134) | H(123-137) |
| 12 | | H(117-131) | H(129-143) | H(141-155) |

### Example 1-3: Preparation of Various Antisera

The immune antigens were prepared by adding 1 mL of PBS to the Eppendorf tubes containing the frozen storage of the four groups of the peptide mixtures prepared in Example 1-2, and then adding an equal amount of Freund's complete adjuvant solution (Fujifilm Wako Pure Chemical Corporation) thereto to create the suspensions. The immunization was conducted by administering the suspensions of the 4 peptide mixtures subcutaneously as an immunogen to two rabbits (New Zealand White Rabbits, weighing 2.5-3.0 kg) every two weeks, with a dose of 50 µg of each peptide per rabbit. A total of five immunizations were conducted, including the initial immunization, and blood was collected from each rabbit one week after the final immunization to obtain the antisera. The anti-peptide sera obtained from Mixed Peptides (10) Dose Group A in the 2 rabbits were named anti-sera A1 and A2, and the anti-peptide sera from Dose Groups B, C, and D were named anti-sera B1 and B2, anti-sera C1 and C2, and anti-sera D1 and D2, respectively.

### Example 1-4: Analysis of Various Antisera

The obtained anti-peptide sera were analyzed as follows.

The peptide solid-phase solution was diluted with DMSO to a concentration of 10 µg/mL, and the negative control BSA and the positive control human TNFα were adjusted to 100 µg/mL in PBS. Each antigen peptide corresponding to Mixed Peptides Dose Groups A to D in 5 µL aliquots was added to a 96-well microplate, then 100 µL of PBS was added thereto, and the mixtures were allowed to stand at room temperature for 2 hours, thereby solid-phasing each antigen peptide. Next, after removing the antigen solution, 400 µL of 0.1% BSA/PBS was added to each well, the plate was covered with an ELISA sheet plate film, and stored at 4°C until use.

The stored antigen plate was washed three times with PBS/0.05% Tween-20. The anti-peptide sera from Mixed Peptides Dose Groups A to D, diluted at 100-fold on this plate, in 100 µL each, were added to wells solid-phased with each antigen peptide, as well as BSA and TNFα, and the plate was incubated overnight at room temperature. Then, the plate was washed three times with PBS/0.05% Tween-20. Next, 100 µL of HRP (horseradish peroxidase)-labeled anti-rabbit IgG antibody (manufactured by Bio-Rad) was added to each well, and the mixtures were incubated at room temperature for 2 hours. Then, the plate was washed three times with PBS/0.05% Tween-20. Finally, 100 µL of 3,3',5,5'-Tetramethylbenzidine (TMB) was added to each well, and after sufficient color development, 100 µL of 1N sulfuric acid solution was added to each well to stop the reaction. The color development of the substrate solution was measured at an absorbance of 450 nm using a plate reader (manufactured by Titertek).

The results are as follows.

### A: Antisera A1 and A2

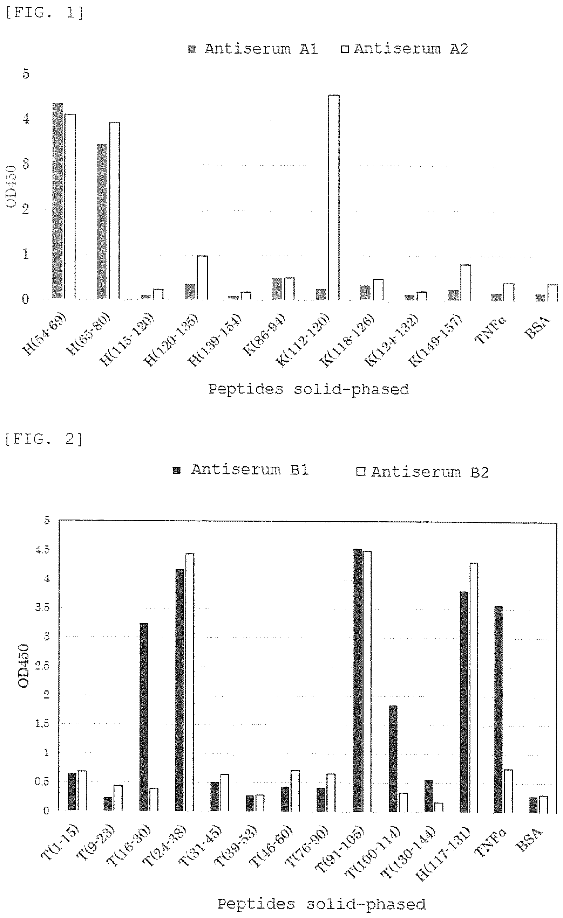

Antisera A1 and A2 diluted at 100-fold were determined for antibody titers using a plate solid-phased with 10 antigen peptides (Mixed Peptides (10) Dose Group A). The results are shown in Table 5 and Figure 1.

**Table 5: Reactivity of Antisera A1 and A2 Against Each Antigen Peptide (OD450)**

| [Table 5] | | | | |
|---|---|---|---|---|
| Antigen peptides | antiserum A1 | | antiserum A2 | |
| | OD450 | S/N ratio | CD450 | S/N ratio |
| H(54-69) | 4.340 | 26.0 | 4.102 | 10.7 |
| H(65-80) | 3.442 | 20.6 | 3.915 | 10.2 |
| H(115-120) | 0.113 | 0.7 | 0.233 | 0.6 |
| H(120-135) | 0.361 | 2.2 | 0.966 | 2.5 |
| H(139-154) | 0.102 | 0.6 | 0.181 | 0.5 |
| K(86-94) | 0.481 | 2.9 | 0.502 | 1.3 |
| K(112-120) | 0.263 | 1.6 | 4.567 | 11.9 |
| K(118-126) | 0.335 | 2.0 | 0.480 | 1.2 |
| K(124-132) | 0.130 | 0.8 | 0.201 | 0.5 |
| K(149-157) | 0.252 | 1.5 | 0.806 | 2.1 |
| TNFα | 0.166 | 1.0 | 0.399 | 1.0 |
| BSA | 0.167 | 1 | 0.385 | 1 |

S/N ratio is a ratio of the antibody titer one week after the last administration to the antibody titer on the starting date of the administration.

As seen in Table 5 and Figure 1, among the 10 peptides administered, the helper peptides H(54-69) and H(65-80) having 16 amino acid residues were confirmed to induce an increase in antibody titer in both antisera, and the killer peptide K(112-130) was observed for the induction of antibodies against only A2. However, the increase in antibody titers against human TNFα could not be confirmed in either case.

### B: Antisera B1 and B2

Antisera B1 and B2 diluted at 100-fold were determined for antibody titers using a plate solid-phased with 12 antigen peptides (Mixed Peptides (12) Dose Group B). The results are shown in Table 6 and Figure 2.

**Table 6: Reactivity of Antisera B1 and B2 Against Each Antigen Peptide (OD450)**

| [Table 6] | | | | |
|---|---|---|---|---|
| Antigen peptides | antiserum B1 | | antiserum B2 | |
| | OD450 | S/N ratio | OD450 | S/N ratio |
| T(1-15) | 0.653 | 2.3 | 0.682 | 2.2 |
| T(9-23) | 0.232 | 0.8 | 0.444 | 1.4 |
| T(16-30) | 3.239 | 11.2 | 0.390 | 1.3 |
| T(24-38) | 4.176 | 14.4 | 4.451 | 14.4 |
| T(31-45) | 0.515 | 1.8 | 0.635 | 2.1 |
| T(39-33) | 0.279 | 1.0 | 0.284 | 0.9 |
| T(46-60) | 0.442 | 1.5 | 0.714 | 2.3 |
| T(76-90) | 0.414 | 1.4 | 0.661 | 2.1 |
| T(91-105) | 4.537 | 15.6 | 4.507 | 14.6 |
| T(100-114) | 1.845 | 6.4 | 0.343 | 1.1 |
| T(130-144) | 0.554 | 1.9 | 0.177 | 0.6 |
| H(117-131) | 3.809 | 13.1 | 4.305 | 13.9 |
| TNFα | 3.570 | 12.3 | 0.742 | 2.4 |
| BSA | 0.290 | 1 | 0.309 | 1 |

S/N ratio is a ratio of the antibody titer one week after the last administration to the antibody titer on the starting date of the administration.

As seen from Table 6 and Figure 2, among the 12 peptides administered, the peptides T(24-38), T(91-105) and H(117-131) having 15 amino acid residues were confirmed to induce an increase in antibody titer in both antisera, and peptides T(16-30) and T(100-114) as well as TNFα were observed for the induction of antibodies against only antiserum B1, while no increase in antibody titer against TNFα was confirmed in antiserum B2.

### C: Antisera C1 and C2

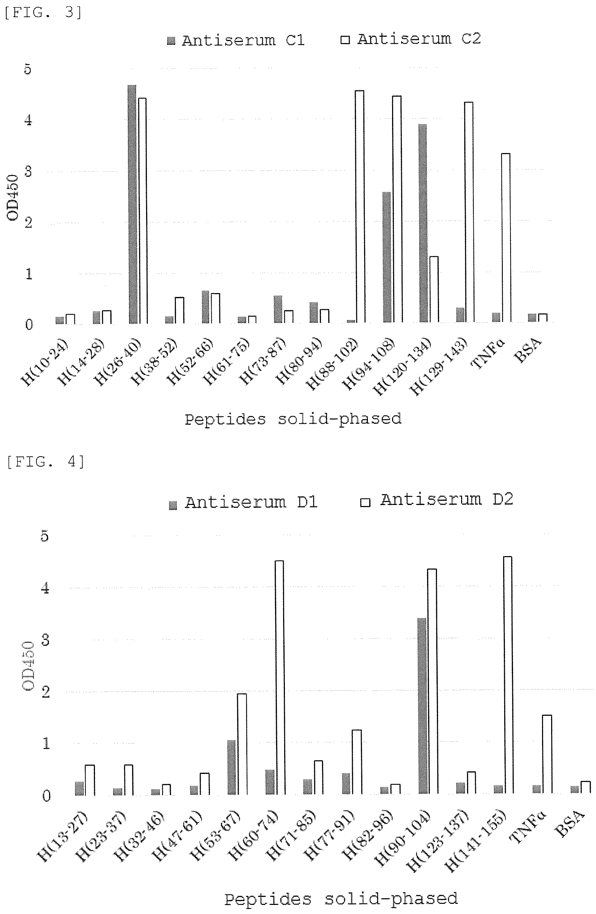

Antisera C1 and C2 diluted at 100-fold were determined for antibody titers using a plate solid-phased with 12 antigen peptides (Mixed Peptides (12) Dose Group C). The results are shown in Table 7 and Figure 3.

**Table 7: Reactivity of Antisera C1 and C2 Against Each Antigen Peptide (OD450)**

| [Table 7] | | | | |
|---|---|---|---|---|
| Antigen pep tides | antiserum C1 | | antiserum C2 | |
| | OD450 | S/N ratio | OD450 | S/N ratio |
| H (10-24) | 0.149 | 0.9 | 0.209 | 1.3 |
| H (14-28) | 0.256 | 1.5 | 0.274 | 1.7 |
| H (26-40) | 4.677 | 26.9 | 4.425 | 27.0 |
| H (38-52) | 0.150 | 0.9 | 0.531 | 3.2 |
| H (52-66) | 0.666 | 3.8 | 0.589 | 3.6 |
| H (61-75) | 0.133 | 0.8 | 0.141 | 0.9 |
| H (73-87) | 0.554 | 3.2 | 0.250 | 1.5 |
| H (80-94) | 0.433 | 2.5 | 0.267 | 1.6 |
| H (88-102) | 0.071 | 0.4 | 4.539 | 27.7 |
| H (94-108) | 2.570 | 14.8 | 4.434 | 27.0 |
| H (120-134) | 3.888 | 22.3 | 1.294 | 7.9 |
| H (129-143) | 0.304 | 1.7 | 4.313 | 26.3 |
| TNFα | 0.187 | 1.1 | 3.299 | 20.1 |
| BSA | 0.174 | 1 | 0.164 | 1 |

As seen in Table 7 and Figure 3, among the 12 peptides administered, the peptides H(26-40) and H(94-108) having 15 amino acid residues were confirmed to indue an increase in antibody titer in both antisera, and peptides H(88-102) and H(129-143) as well as TNFα were observed for the induction of antibodies against only antiserum C1, while no increase in antibody titer against TNFα was confirmed in antiserum C2.

### D: Antisera D1 and D2

Antisera D1 and D2 diluted at 100-fold were determined for antibody titers using a plate solid-phased with 12 antigen peptides (Mixed Peptides (12) Dose Group D). The results are shown in Table 8 and Figure 4.

**Table 8: Reactivity of Antisera D1 and D2 Against Each Antigen Peptide (OD450)**

| [Table 8] | | | | |
|---|---|---|---|---|
| Antigen peptides | antiserum D1 | | antiserum D2 | |
| | OD450 | S/N ratio | OD450 | S/N ratio |
| H (13-27) | 0.263 | 1.8 | 0.594 | 2.6 |
| H (23-37) | 0.134 | 0.9 | 0.579 | 2.5 |
| H (32-46) | 0.123 | 0.8 | 0.196 | 0.8 |
| H (47-61) | 0.184 | 1.3 | 0.415 | 1.8 |
| H (53-67) | 1.064 | 7.3 | 1.943 | 8.4 |
| H (60-74) | 0.484 | 3.3 | 4.501 | 19.4 |
| H (71-85) | 0.288 | 2.0 | 0.645 | 2.8 |
| H (77-91) | 0.407 | 2.8 | 1.237 | 5.3 |
| H (82-96) | 0.130 | 0.9 | 0.185 | 0.8 |
| H (90-104) | 3.380 | 23.2 | 4.335 | 18.7 |
| H (123-137) | 0.217 | 1.5 | 0.425 | 1.8 |
| H (141-155) | 0.163 | 1.1 | 4.566 | 19.7 |
| TNFα | 0.152 | 1.0 | 1.499 | 6.5 |
| BSA | 0.146 | 1 | 0.232 | 1 |

As seen from Table 8 and Figure 4, among the 12 peptides administered, the 15AA peptides H(53-67) and H(90-104) were confirmed to induce an increase in antibody titer in both antisera, and the peptides H(60-74) and H(141-155) as well as TNFα were observed for the induction of antibodies against only antiserum D2, while no increase in antibody titer against TNFα was confirmed in antiserum D1.

### Example 2

### Evaluation of reactivity of anti-serum obtained by subcutaneously immunizing rabbits with hTNFα peptide mixtures against hTNFα

In Example 1, the hTNFα peptide mixtures were used to measure the antibody titer of the rabbit antisera. In this example, each antiserum diluted to eight different dilution solutions was detected for reactivity with TNFα to reconfirm that the pattern of the antigen-antibody reaction was specific.

The human TNFα (hTNFα) adjusted to 100 µg/mL in PBS was added in 5 µL to each well of a 96-well microplate, then additionally, 100 µL of PBS was added thereto, and the mixture was allowed to stand at room temperature for 2 hours, thereby solid-phasing the human TNFα. Next, after removing the antigen solution, 400 µL of 0.1% BSA/PBS was added to each well, the plate was covered with an ELISA sheet plate film, and stored at 4°C until use.

The eight antisera A1 to D2 prepared in Example 1-3 were diluted at 100-fold with 0.1% BSA/PBS, and further diluted at 3-fold with 0.1% BSA/PBS by seven steps. Similarly, the anti-human TNFα rabbit serum and the normal rabbit serum (NRS: Normal Rabbit Serum) as a control serum were also diluted.

The stored antigen plate was washed three times with PBS/0.05% Tween-20, and 100 µL each of the diluted 8 antisera A1 to D2 was added to a well solid-phased with TNFα, followed by incubating the same overnight at room temperature. Then, the plates were washed three times with PBS/0.05% Tween-20. Next, 100 µL of HRP (horseradish peroxidase) -labeled anti-rabbit IgG antibody (manufactured by Bio-Rad) was added to each well, and the mixtures were incubated at room temperature for 2 hours. Then, the plate was washed three times with PBS/0.05% Tween-20. Finally, 100 µL of 3,3', 5,5'-Tetramethylbenzidine (TMB) was added to each well, and after sufficient color development, 100 µL of 1N sulfuric acid solution was added to each well to stop the reaction. The color development of the substrate solution was measured at an absorbance of 450 nm using a plate reader (manufactured by Titertek).

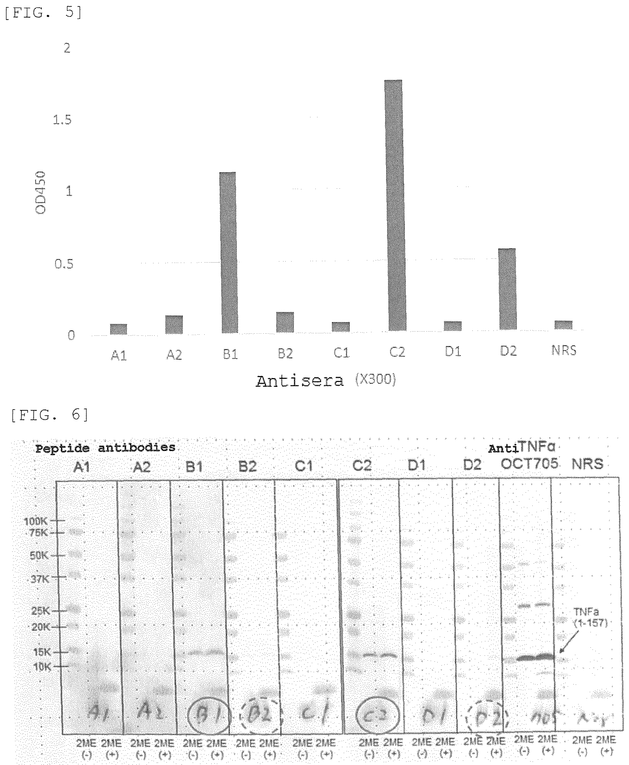

The results obtained are shown in Table 9 and Figure 5. These indicate that antibodies against TNFα were confirmed in the antisera B1, C2, and D2.

**Table 9: Reactivity of Eight Antisera to Human TNFα (OD450)**

| [Table 9] | | | | | | | | | |
|---|---|---|---|---|---|---|---|---|---|
| Diluti on | A1 | A2 | B1 | B2 | C1 | C2 | D1 | D2 | NRS |
| X100 | 0.093 | 0.485 | 1.293 | 0.428 | 0.166 | 2.36 | 0.096 | 1.229 | 0.073 |
| X300 | 0.079 | 0.135 | 1.125 | 0.146 | 0.075 | 1.751 | 0.071 | 0.569 | 0.062 |
| X900 | 0.06 | 0.081 | 0.483 | 0.082 | 0.057 | 0.893 | 0.057 | 0.218 | 0.051 |
| X2700 | 0.049 | 0.067 | 0.192 | 0.056 | 0.049 | 0.398 | 0.05 | 0.102 | 0.048 |
| X8100 | 0.047 | 0.055 | 0.099 | 0.051 | 0.047 | 0.184 | 0.047 | 0.064 | 0.045 |
| X24300 | 0.044 | 0.049 | 0.064 | 0.046 | 0.045 | 0.092 | 0.045 | 0.05 | 0.046 |
| X72900 | 0.045 | 0.046 | 0.052 | 0.046 | 0.044 | 0.063 | 0.045 | 0.048 | 0.045 |
| X21870 0 | 0.043 | 0.045 | 0.047 | 0.045 | 0.045 | 0.05 | 0.045 | 0.044 | 0.043 |

### Example 3

### Identification of peptides that induce antibodies against human TNFα

The antibodies against the human TNFα were confirmed in three antisera: B1, C2, and D2, and therefore, among them, antisera B1 and C2, which had higher antibody titers, were examined for peptides that induce the antibodies against human TNFα.

The method involved adding the mixed peptides as used in antisera B1 and C2 individually to a system where human TNFα and an antiserum diluted moderately were reacted, and identifying the peptides that inhibit the binding of human TNFα to the antisera.

### Example 3-1: In the case of antiserum B1

The 12 peptides of Mixed Peptides (12) Dose Group B were diluted with 0.1% BSA/PBS to a concentration of 60 pg/mL, and 50 µL aliquot each of them was added to a 96-well plate solid-phased with human TNFα (Example 1-4) (Wells With Peptides), while, as a control, 50 µL aliquot of 0.1% BSA/PBS was added (Wells Without Peptides), followed by adding 100 µL of antiserum B1, which had been diluted at 100-fold with 0.1% BSA/PBS, and incubating the plate at room temperature overnight. After this, the plates were washed three times with PBS/0.05% Tween-20. Next, 100 µL of HRP (horseradish peroxidase)-labeled anti-rabbit IgG antibody (manufactured by Bio-Rad) was added to each well, and the mixtures were incubated at room temperature for 2 hours. Then, the plate was washed three times with PBS/0.05% Tween-20. Finally, 100 µL of 3,3',5,5'-Tetramethylbenzidine (TMB) was added to each well, and after sufficient color development, 100 µL of 1N sulfuric acid solution was added to each well to stop the reaction. The color development of the substrate solution was measured at an absorbance of 450 nm using a plate reader (manufactured by Titertek). The inhibition rate % was calculated from the measurement values of Wells with Peptides (+) and Wells without Peptide (-).

The results are shown in Table 10. Table 10 shows that peptide T(24-38) and peptide T(31-45) inhibited the binding of antiserum B1 to human TNFα by more than 90%, and on the other hand, Table 6 shows that the five peptides: T(16-30), T(24-38), T(91-105), T(100-114), and H(117-131) induced peptide antibodies in antiserum B1. The fact that, no antibody induction was confirmed in T(31-45), which had over 90% inhibition, indicated that the peptide antibody derived from peptide T(24-38) bound to human TNFα, and this binding was inhibited by peptide T(24-38) and peptide T(31-45).

**Table 10: Peptides that inhibit the binding of antiserum B1 to human TNFα**

| [Table 10] | | | |
|---|---|---|---|
| Added peptides | Without (-) | With (+) | inhibition rate (%) |
| T(1-15) | 1.256 | 1.098 | 13 |
| T(9-23) | 1.112 | 1.064 | 4 |
| T(16-30) | 1.165 | 1.056 | 9 |
| T(24-38) | 1.318 | 0.103 | 92 |
| T(31-45) | 1.244 | 0.106 | 91 |
| T(39-53) | 1.303 | 1.007 | 23 |
| T(46-60) | 1.287 | 1.159 | 10 |
| T(76-90) | 1.291 | 1.120 | 13 |
| T(91-105) | 1.207 | 1.173 | 3 |
| T(100-114) | 1.237 | 1.062 | 14 |
| H(117-131) | 1.207 | 1.203 | 0 |
| T(130-144) | 1.223 | 1.108 | 9 |

### Example 3-2: In the case of antiserum C2

According to a similar method to antiserum B1, a peptide that inhibits the binding of human TNFα was identified from Mixed Peptides (12) Dose Group C.

The results of antiserum C2 are shown in Table 11. Table 11 shows that peptide H(26-40) inhibited the binding of antiserum C2 to human TNFα by over 80%, and Table 7 shows that the five peptides: H(26-40), H(88-102), H(94-108), H(120-134), and H(129-143) induced peptide antibodies in antiserum C2. The fact that peptide H(26-40), which had over 80% inhibition, had been confirmed to induce peptide antibodies, indicates that the peptide antibody derived from peptide H(26-40) bound to TNFα, and this binding was inhibited by peptide H(26-40).

**Table 11: Peptides that inhibit the binding of antiserum C2 to human TNFα**

| Table 11] | | | |
|---|---|---|---|
| Added peptides | Without (-) | With (+) | inhibition rate (%) |
| H(10-24) | 1.879 | 1.735 | 8 |
| H(14-28) | 1.777 | 1.645 | 7 |
| H(26-40) | 1.743 | 0.313 | 82 |
| H(38-52) | 1.765 | 1.422 | 19 |
| H(52-66) | 1.708 | 1.346 | 21 |
| H(61-75) | 1.916 | 1.729 | 10 |
| H(73-87) | 1.78 | 1.795 | -1 |
| H(80-94) | 1.94 | 1.698 | 12 |
| H(88-102) | 1.869 | 1.777 | 5 |
| H(94-108) | 1.903 | 1.786 | 6 |
| H(120-134) | 1.957 | 1.769 | 10 |
| H(129-143) | 2.02 | 1.781 | 12 |

The results as shown above indicated that peptide antibodies derived from T(24-38) bound to human TNFα in antiserum B1, while peptide antibodies derived from H(26-40) bound to human TNFα in antiserum C2. In this regard, it would be possible that the inhibitions by the peptides were not 100% due to either i) an insufficient amount of the added peptides, or ii) responsibility of any other peptide for the inhibition, even with a sufficient amount of the added peptides. In the latter case, it would be necessary to perform similar operations or repeat them to find the other sequence after removing the peptide antibodies of T(24-38) and H(26-40) from the antisera using peptide T(24-38) and peptide H(26-40) columns.

### Example 4

### Confirmation of antibody-inducing ability of peptide T(24-38) identified in Example 3 against human TNFα

Next, it was confirmed whether the peptides derived from the peptide inhibition test induced antibodies against human TNFα.

Peptide T(24-38) obtained from antiserum B1 as identified in Example 3 was selected, and the administration conditions were set for: i) increasing the number of rabbits from 2 to 10, ii) increasing the dose of the peptide amount from 0.05 mg per rabbit to 1 mg per rabbit, and iii) unifying the adjuvant to incomplete Freund's adjuvant. The immunization was conducted via subcutaneous administration every two weeks, and blood was drawn one week after the fourth administration, followed by measuring the antibody titer. The antibody titer was expressed as S/N ratio in which the OD450 value of a 100-fold dilution of the collected serum was divided by the OD450 value of a 100-fold dilution of the serum collected before peptide administration.

The results are shown in Table 12. The antibody titers against TNFα in ten rabbits administered with peptide T(24-38) varied, but the S/N ratio (a ratio of specific binding to non-specific binding) ranged from 4.7 to 35.7, and all rabbits achieved the criterion for antibody positivity, which is an S/N ratio of 2 or higher. Therefore, it was demonstrated that the administration of peptide T(24-38) induced and produced antibodies against the derived protein,

### TNFα.

The measurement of the antibody titer was conducted in accordance with Example 1-4.

**Table 12: Antibody Titers in Serum of Rabbits Administered with peptide T(24-38) (at 100-fold Dilution)**

| [Table 12] | | | | |
|---|---|---|---|---|
| Individual Nos | antibody titers against H(24-38) | | antibody titers against TNFα | |
| | OD450 | S/N Ratio | OD450 | S/N Ratio |
| 1 | 0.723 | 4.7 | 1.258 | 10.2 |
| 2 | 4.410 | 28.8 | 0.581 | 4.7 |
| 3 | 1.229 | 8.0 | 0.484 | 3.9 |
| 4 | 5.458 | 35.7 | 3.548 | 28.8 |
| 5 | 4.807 | 31.4 | 1.355 | 11.0 |
| 6 | 4.157 | 27.2 | 3.290 | 26.8 |
| 7 | 4.193 | 27.4 | 1.806 | 14.7 |
| 8 | 5.349 | 35.0 | 1.355 | 11.0 |
| 9 | 4.843 | 31.7 | 3.161 | 25.7 |
| 10 | 4.410 | 28.8 | 3.355 | 27.3 |

### Example 5

### Estimation of concentration of antibody against human TNFα in antisera B1 and C2

The antibody titers were presented as OD450 values and SN ratios at a 100-fold dilution. In this example wherein the concentration of antibody against TNFα was estimated, the concentrations of antibodies in antisera B1 and C2 against human TNFα were examined using the therapeutic drug Humira (anti-human TNFα antibody) as a standard.

The details are as follows. Both Humira and the anti-TNFα antibodies in rabbit-administered sera can bind to human TNFα, but the species-specific HRP anti-IgG for humans and rabbits cannot be used on the same plate. Therefore, a horseradish peroxidase (HRP) labeled form of Protein G, which strongly reacted with human IgG and rabbit IgG, was used in this example.

The standard samples of Humira (0, 31, 63, 125, 250, 500, 1000, 2000 ng/mL) were added to the plate solid-phased with human TNFα, and they were diluted by 100- to 2-fold to prepare eight different dilution solutions. Aliquots of 100 µL of the diluted antisera B1 and C2 were added thereto, and incubated at room temperature overnight. Then, the plates were washed three times with PBS/0.05% Tween-20. Next, 100 µL of HRP (horseradish peroxidase)-labeled anti-rabbit IgG antibody (manufactured by Bio-Rad) was added to each well, and the mixtures were incubated at room temperature for 2 hours. Then, the plate was washed three times with PBS/0.05% Tween-20. Finally, 100 µL of 3,3',5,5'-Tetramethylbenzidine (TMB) was added to each well, and after sufficient color development, 100 µL of 1N sulfuric acid solution was added to each well to stop the reaction. The color development of the substrate solution was measured at an absorbance of 450 nm using a plate reader (manufactured by Titertek). The concentrations of the antibodies against TNFα in antisera B1 and C2 were determined from the standard, Humira.

As a result, the amounts of TNF antibodies in B1 and C2 were 22.5 µg/mL and 22.7 µg/mL in terms of Humira, which were considered to represent approximately 0.023% of the total IgG amount in the rabbit, estimated at 10 mg/mL. As a method of direct measurement, it is also possible to perform affinity purification using a peptide column and calculate the recovery rate.

### Example 6

### Evaluation of reactivity of antisera obtained by subcutaneously immunizing rabbits with human TNFα peptides against human TNFα by Western blotting

The evaluation method involved five stages: sample preparation, electrophoresis, transfer, blocking, and antibody reaction.

### Sample preparation:

100 µL of a 10 µg/mL human TNFα solution was mixed with 100 µL of the dissolution buffer (2ME-/+) and the mixture was heated at 95°C for 5 minutes in a heat block.

### Electrophoresis:

Ten µL of heat-treated samples (human TNFα solutions) was applied per lane to the 12.5% SDS-PAGE gel, and electrophoresis was performed at 20 mA for approximately 1 hour per gel.

### Transfer:

The nitrocellulose membrane and two sheets of filter paper were pre-soaked in the transfer solution (25 mM Tris, 192 mM glycine, 10% MeOH), and then they were sandwiched on both sides of the gel after electrophoresis, followed by mounting the same in the semi-dry blotting apparatus. The condition for the transfer was a 30-minute electrification at 25 V.

### Blocking:

The nitrocellulose membrane after the transfer was immersed in the blocking solution (1% skim milk/PBS) and agitated at room temperature for 30 minutes.

### Antibody reaction:

The eight antisera A1 to D2 prepared in Example 1-3 and the control sera (anti-human TNFα rabbit serum and normal rabbit serum) were diluted at 1000-fold with 0.1% BSA/PBS/0.1% Tween 20. The cut nitrocellulose membrane was placed into a nylon bag, and various types of antiserum diluents, anti-human TNFα rabbit serum and normal rabbit serum solutions were added thereto, followed by allowing the same to react overnight at 4°C. Next, the antibody was discarded, and 50 mL of 0.05% Tween/PBS was added thereto, followed by shaking the same for 5 minutes. This washing operation was repeated more than three times. The secondary antibody labeled with HRP diluted at 1000-fold with 0.05% Tween/PBS/0.1% BSA was added to the nitrocellulose membrane, and the sample was agitated at room temperature for 30 minutes. The HPR-labeled anti-rabbit solution was discard, and the nitrocellulose membrane was similarly washed, followed by developing a color in the same under the 4-chloro-1-naphthol/10% methanol solution.

The results of examining the binding of antisera A1 to D2 to human TNFα using Western blotting are shown in Figure 6. As shown in Figure 6, antisera B1 and C2 reacted strongly with human TNFα, while antisera B2 and D2 exhibited a weak reaction. The results were consistent with the results of the antibody titer against TNFα using the solid-phased human TNFα in Example 2. Further, no difference in reactivity before and after the treatment of TNFα with 2ME was recognized. This means that even when the disulfide bonds within the TNFα are cleaved by 2ME, the reactivity of the antisera does not change, indicating that antisera A1 to D2 recognize the primary structure of TNFα rather than its secondary structure. The Western blot analysis was expected to yield different results compared to the analysis of the external recognition antibodies for TNFα, which are recognized by the ELISA, since the former disrupts the three-dimensional structure of TNFα with SDS and +2ME, exposing the internal peptides and enhancing the reactivity of the peptide antibodies.

### Example 7

### Measurement of antibody titers in antisera obtained by subcutaneously immunizing rabbits with mixtures of 15 human TNFα peptides

The human TNFα peptide mixtures E to H were prepared by weighing each human TNFα peptide to achieve the composition shown in Table 13 and mixing them in equal amounts (weight ratio).

**Table 13: Compositions of various mixtures of human TNFα (hTNFα) peptides**

| [Table 13] | | | |
|---|---|---|---|
| mixture E | mixture F | mixture G | mixture H |
| hTNFα(1-15) (SEQ ID NO:11) | hTNFα(16-30) (SEQ ID NO:13) | hTNFα(26-40) (SEQ ID NO:26) | hTNFα (39-53) (SEQ ID NO: 16) |
| hTNFα (54-69) (SEQ ID NO:1) | hTNFα (60-74) (SEQ ID NO:32) | hTNFα(53-67) (SEQ ID NO:31) | hTNFα (71-85) (SEQ ID NO: 34) |
| hTNFα(100-114) (SEQ ID NO:20) | hTNFα (115-130) (SEQ ID NO: 3) | hTNFα (73-87) (SEQ ID NO:35) | hTNFα(88-102) (SEQ ID NO: 39) |
| hTNFα(142-150) LDFAESGQV (SEQ ID NO:52) | | hTNFα(129-143) (SEQ ID NO: 45) | hTNFα(143-157) DFAESGQVYFGIIAL (SEQ ID NO: 53) |

Emulsions of Human TNFα peptide mixture F at 0.3 mg/rabbit, and human TNFα peptide mixtures E, G, and H at 0.4 mg/rabbit (0.1 mg of each peptide per rabbit) mixed in equal amounts with complete/incomplete Freund's adjuvant were administered subcutaneously once at two-week intervals in a total of four times into rabbits. Blood samples were collected on the date of the start of administration and one week after the final administration, obtaining antisera E to H against peptide mixtures E to H. The antibody titers of antisera E to H against each human TNFα peptide, as well as the antibody titer against human TNFα, were measured using the ELISA. The results were calculated as the ratio of the antibody titer one week after the final administration to the antibody titer on the date of the start of administration (S/N ratio).

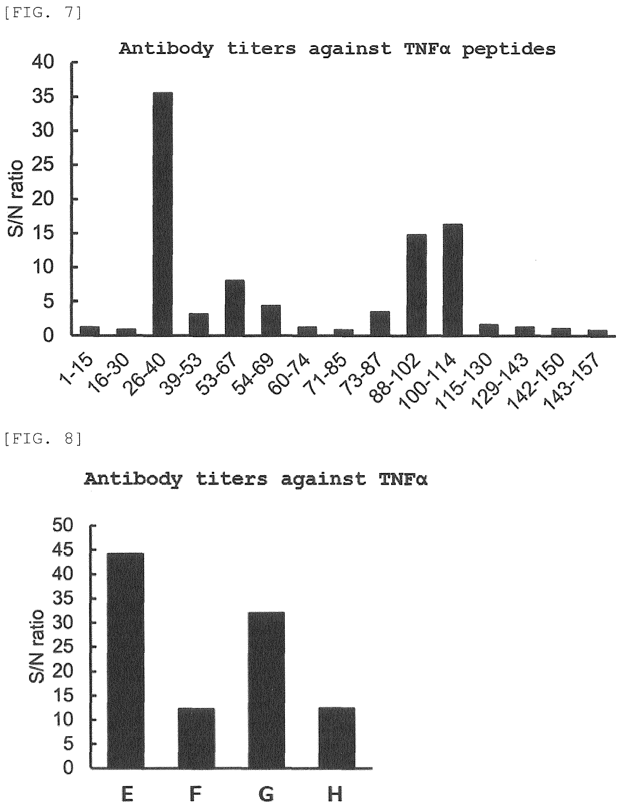

The antibody titers of antisera E to H against each human TNFα peptide, specifically the antibody titers of antiserum E against each human TNFα peptide in mixture E, the antibody titers of antiserum F against each human TNFα peptide in mixture F, the antibody titers of antiserum G against each human TNFα peptide in mixture G, and the antibody titers of antiserum H against each human TNFα peptide in mixture H, are presented in a single graph, which is as shown in Figure 7. Further, the antibody titers of antisera E to H against human TNFα are shown in Figure 8. Figure 7 shows that the subcutaneous administration of the human TNFα peptide mixtures combined with an adjuvant resulted in an increase in several anti-human TNFα peptide antibody titers. Further, Figure 8 shows that the subcutaneous administration of any of the human TNFα peptide mixtures also resulted in an increase in the antibody titer against the derived protein, human TNFα. From the above, it was demonstrated that the combination of the peptide mixtures and the adjuvant was subcutaneously administered to rabbits facilitates to induce antibodies that can recognize the peptides or the derived protein.

### Example 8

### Measurement of antibody titer of antisera obtained from subcutaneous immunization with human TNFα peptide alone

Referring to the human TNFα peptides that induced antibodies (Figure 7) in Example 7, the antibody titer of the antiserum obtained by administering the human TNFα peptide alone was measured.

Specifically, according to the identification of the peptide that induces the antibody against human TNFα in Example 3, hTNFα(100-114) was identified in antiserum E, hTNFα(26-40) was identified in antiserum G, and hTNFα(143-157) was identified in antiserum H. The reason for selecting human TNFα (143-157) in antiserum H was based on the fact that the C-terminus of the TNF-α trimeric higher-order structure was exposed to the exterior in solution, and that the C-terminus was also responsible for binding to the TNF-α receptor.

Emulsions of hTNFα(26-40), hTNFα(100-114), or hTNFα(143-157) alone at 1 mg/rabbit mixed in equal amounts with complete/incomplete Freund's adjuvant were administered subcutaneously once at two-week intervals in a total of four times into rabbits. Blood samples were collected on the date of the start of administration and one week after the final administration, obtaining the antisera. The antibody titers against human TNFα were measured using the ELISA. The results were calculated as the ratio of the antibody titer one week after the final administration to the antibody titer on the date of the start of administration (S/N ratio) .

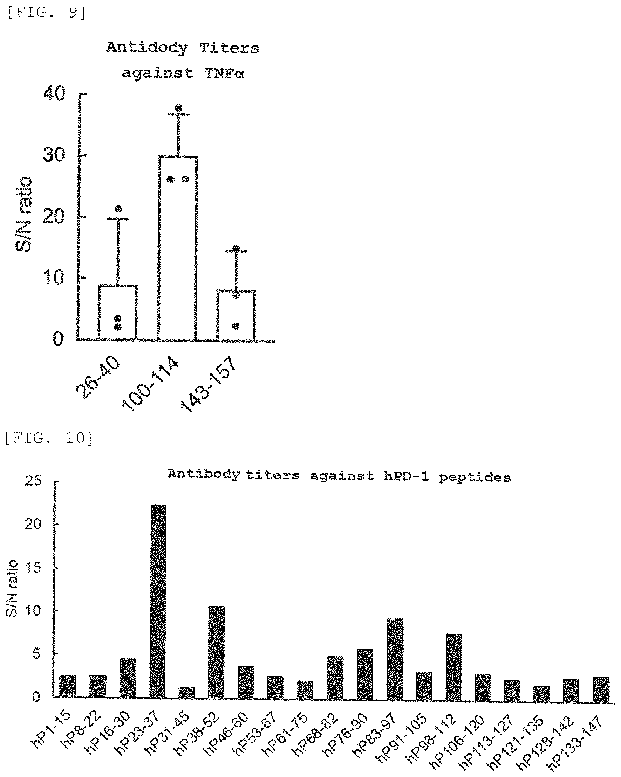

The results are shown in Figure 9. Figure 9 shows that the subcutaneous administration of the human TNFα peptide alone combined with an adjuvant resulted in an increase in the antibody titer against the derived protein, human TNFα. From the above, it was demonstrated that not only the peptide mixture but also the peptide alone combined with the adjuvant could induce antibodies that recognized the derived protein when administered subcutaneously.

### Example 9

### Measurement of antibody titers of antisera obtained by subcutaneously immunizing rabbits with human B-amyloid peptide

Five peptides in total, which were peptide 1 (1-14), peptide 3 (15-28), and peptide 5 (29-42) that were obtained by dividing the 42 of the full-length amino acid sequence into three parts, as well as peptide 2 (8-21) and peptide 4 (22-35) overlapping among the peptide, were synthesized by Genzyme (Table 14).

### Table 14

**[Table 14]**

| Solid-phased antigens | Positions | Amino acid sequences |
|---|---|---|
| peptide 1 | 1-14 | DAEFRHDSGYEVHH (SEQ ID NO: 47) |
| peptide 2 | 8-21 | QKLVFFAEDVGSNK (SEQ ID NO: 48) |
| peptide 3 | 15-28 | GAIIGLMVGGVVIA (SEQ ID NO: 49) |
| peptide 4 | 22-35 | SGYEVHHQKLVFFA (SEQ ID NO: 50) |
| peptide 5 | 29-42 | EDVGSNKGAIIGLM (SEQ ID NO: 51) |

Samples in 0.1 mL of each of the five synthetic peptides dissolved in dimethyl sulfoxide (DMSO) at a concentration of 10 mg/mL were placed into a 1.5 mL Eppendorf tube, mixed, and the resulting peptide mixtures were aliquoted into Eppendorf tubes in 0.05 mL portions, followed by conducting cryopreservation of the same.

One mL of PBS was added to the preserved Eppendorf tube, and then an equal amount of Freund's complete adjuvant was added thereto to prepare a suspension as an immunogen. The suspension was subcutaneously administered to the rabbit (New Zealand White Rabbit, weighing 2.5-3.0 kg) as an immunogenic antigen at a dose of 100 pg of each peptide per rabbit, every two weeks, thereby conducing the immunization. A total of five immunizations were conducted, including the initial immunization, and blood samples were collected from each rabbit one week after the final immunization to obtain the anti-peptide rabbit serum.

The measurement of the antibody titer of the antiserum was conducted as follows.

### Preparation of antigen plates:

The five peptides were diluted in DMSO to a concentration of 10 µg/mL, and the negative control BSA was adjusted to 100 µg/mL with PBS. Each antigen was added in 5 µL to each well of a 96-well microplate, then additionally, 100 µL of PBS was added thereto, and the mixture was allowed to stand at room temperature for 2 hours, thereby solid-phasing the human TNFα. Next, after removing the antigen solution, 400 µL of 0.1% BSA/PBS was added to each well, the plate was covered with an ELISA sheet plate film, and stored at 4°C until use.

### Dilution of antiserum:

The anti-peptide rabbit serum previously prepared were diluted with 0.1% BSA/PBS at 1000-fold, and then perform a further 3-fold dilution by seven steps to prepare eight different dilution solutions: 1000 (1K)-fold, 3K-fold, 9K-fold, 27K-fold, 81K-fold, 243K-fold, 729K-fold, and 2187K-fold.

### Measurement of antibody titer:

The stored antigen plate was washed three times with PBS/0.05%/ Tween-20 with 0.1%, and 100 µL of the anti-peptide rabbit serum diluted in the stepwise diluted solution was added to a plate solid-phased with the five peptides and BSA, followed by incubating the plate at room temperature overnight, and washing the same three times with PBS/0.05% Tween-20. Next, 100 µL of HRP (horseradish peroxidase)-labeled anti-rabbit IgG antibody (manufactured by Bio-Rad) was added to each well, and the mixtures were incubated at room temperature for 2 hours. Then, the plate was washed three times with PBS/0.05% Tween-20. Finally, 100 µL of 3,3',5,5'-Tetramethylbenzidine (TMB) was added to each well, and after sufficient color development, 100 µL of 1N sulfuric acid solution was added to each well to stop the reaction. The color development of the substrate solution was measured at an absorbance of 450 nm using a plate reader (manufactured by Titertek).

The antibody titers (OD450) of the 3000-fold diluted anti-peptide rabbit sera against the five peptides and BSA are shown in Table 15.

### Table 15

**[Table 15]**

| Solid-phased antigens | Antibody titers (OD450) | Peptide /BSA |
|---|---|---|
| peptide 1 | 2.27 | 8.7 |
| peptide 2 | 0.64 | 2.5 |
| peptide 3 | 1. 67 | 6. 4 |
| peptide 4 | 3.72 | 14.3 |
| peptide 5 | 0.28 | 1.1 |
| BSA | 0.26 | 1 |

The peptide/BSA shows the relative antibody titers of the five peptides, with the color development of the negative control BSA set to 1. peptides 1, 3, and 4 indicate a OD450 = 1 or higher, with peptide 4 showing particularly high antibody titers. On the other hand, peptide 5 showed almost the same color development as the negative control BSA. This indicates that humans and rabbits possess the same β-amyloid sequence, and that the administration of peptide 1 (1-14), peptide 3 (15-28), and peptide 4 (22-35) can confirm the antibody induction, particularly showing that peptide 4 (22-35) has a high antibody induction capability.

### Example 10

### Measurement of antibody titers of antisera obtained from subcutaneous immunization with human PD-1 peptide mixtures

Various human PD-1 peptide mixtures were prepared by weighing each human PD-1 peptide (hPD-1 peptide) to achieve the composition shown in Table 16 and mixing them in equal amounts (weight ratio).

**Table 16: Compositions of various mixtures of human PD-1 peptides**

| [Table 16] | | | |
|---|---|---|---|
| mixture A2 | mixture B2 | mixture C2 | mixture D2 |
| hPD-1(1-15) FLDSPDRPWNPPT FS (SEQ ID NO:68) | hPD-1(16-30) PALLVVTEGDNAT FT (SEQ ID NO:73) | hPD-1(8-22) PWNPPTFSPALLV VT (SEQ ID NO:79) | hPD-1(23-37) EGDNATFTCSFSN TS (SEQ ID NO:84) |
| hPD-1(31-45) CSFSNTSESFVLN WY (SEQ ID NO:69) | hPD-1(46-60) RMSPSNQTDKLAA FP (SEQ ID NO:74) | hPD-1(38-52) ESFVLNWYRMSPS NQ (SEQ ID NO:80) | hPD-1(53-67) TDKLAAFPEDRSQ PG (SEQ ID NO:85) |
| hPD-1(61-75) EDRSQPGQDCRFR VT (SEQ ID NO:70) | hPD-1(76-90) QLPNGRDFHMSVV RA (SEQ ID NO:75) | hPD-1(68-82) QDCRFRVTQLPNG RD (SEQ ID NO:81) | hPD-1(83-97) FHMSVVRARRNDS GT (SEQ ID NO:86) |
| hPD-1(91-105) RRNDSGTYLCGAI SL (SEQ ID NO:71) | hPD-1(106-120 ) APKAQIKESLRAE LR (SEQ ID NO:76) | hPD-1(98-112) YLCGAISLAPKAQ IK (SEQ ID NO:82) | hPD-1(113-127 ) ESLRAELRVTERR AE (SEQ ID NO:87) |
| hPD-1(121-135 ) VTERRAEVPTAHP SP (SEQ ID NO:72) | hPD-1(133-147 ) PSPSPRPAGQFQT LV (SEQ ID NO:78) | hPD-1(128-142 ) VPTAHPSPSPRPA GQ (SEQ ID NO:83) | |

Emulsions of each mixture at 0.4 mg/rabbit, or at 0.5 mg/rabbit (0.1 mg of each peptide per rabbit) mixed in equal amounts with complete/incomplete Freund's adjuvant were administered subcutaneously once at two-week intervals in a total of four times into rabbits. Blood samples were collected on the date of the start of administration and one week after the final administration, obtaining the antisera in a toral number of 4 against mixtures A2, B2, C2, and D2. The antibody titers against each human PD-1 peptide were measured using the ELISA. The experimental method involved sold-phasing each peptide shown in Table 16 one by one as an antigen on the ELISA plate, adding the antisera obtained from mixtures A2, B2, C2, or D2 thereto. When a reaction occurred, it was determined that the peptide in the respective mixture induced the antibody. The results were calculated as the ratio of the antibody titer one week after the final administration to the antibody titer on the date of the start of administration (S/N ratio).

The results are shown in Figure 10. Figure 10 shows that the subcutaneous administration of human PD-1 peptide mixtures combined with an adjuvant resulted in an increase in several anti-human PD-1 peptide antibody titers. From the above, it was demonstrated that, in addition to human TNFα, the combination of the peptide mixtures and the adjuvant was subcutaneously administered to rabbits facilitates to induce antibodies that can recognize the peptides or the derived protein.

### Example 11

### Measurement of antibody titers of antisera obtained from subcutaneous immunization with mouse PD-1 peptide mixtures

Next, Various mouse PD-1 peptide (mPD-1 peptide) mixtures were prepared by weighing each mPD-1 peptide to achieve the composition shown in Table 17 and mixing them in equal amounts (weight ratio).

### Table 17

**[Table 17]**

| mixture A3 | mixture B3 | mixture C3 | mixture D3 |
|---|---|---|---|
| mPD-1(1-15) MWVRQVPWSFTWAVL (SEQ ID NO:128) | mPD-1(15-29) LQLSWQSGWLLEVPN (SEQ ID NO:134) | mPD-1(8-22) WSFTWAVLQLSWQSG (SEQ ID NO:140) | mPD-1(22-36) GWLLEVPNGPWRSLT (SEQ ID NO:55) |
| mPD-1(29-43) NGPWRSLTFYPAWLT (SEQ ID NO:129) | mPD-1(43-57) TVSEGANATFTCSLS (SEQ ID NO:135) | mPD-1(36-50) TFYPAWLTVSEGANA (SEQ ID NO:141) | mPD-1(50-64) ATFTCSLSNWSEDLM (SEQ ID NO:56) |
| mPD-1(57-71) SNWSEDLMLNWNRLS (SEQ ID NO:130) | mPD-1(71-85) SPSNQTEKQAAFCNG (SEQ ID NO:136) | mPD-1(64-78) MLNWNRLSPSNQTEK (SEQ ID NO:142) | mPD-1(78-92) KQAAFCNGLSQPVQD (SEQ ID NO:57) |
| mPD-1(85-99) GLSQPVQDARFQIIQ (SEQ ID NO:131) | mPD-1(99-113) QLPNRHDFHMNILDT (SEQ ID NO:137) | mPD-1(92-106) DARFQIIQLPNRHDF (SEQ ID NO:143) | mPD-1(106-120) FHMNILDTRRNDSGI (SEQ ID NO:58) |
| mPD-1(113-127) TRRNDSGIYLCGAIS (SEQ ID NO:132) | mPD-1(127-141) SLHPKAKIEESPGAE (SEQ ID NO:138) | mPD-1(120-134) IYLCGAISLHPKAKI (SEQ ID NO:144) | mPD-1(134-148) IEESPGAELVVTERI (SEQ ID NO:145) |
| mPD-1(141-155) ELVVTERILETSTRY (SEQ ID NO:133) | mPD-1(155-169) YPSPSPKPEGRFQGM (SEQ ID NO:139) | mPD-1(148-162) ILETSTRYPSPSPKP (SEQ ID NO:145) | |

Emulsions of each mixture at 0.5 mg/rabbit, or at 0.6 mg/rabbit (0.1 mg of each peptide per rabbit) mixed in equal amounts with complete/incomplete Freund's adjuvant were administered subcutaneously once at two-week intervals in a total of four times into rabbits. Blood samples were collected on the date of the start of administration and one week after the final administration, obtaining the antisera in a toral number of 4 against mixtures A, B, C, and D. The antibody titers against each mPD-1 peptide were measured using the ELISA. The experimental method involved sold-phasing each peptide (mPD-1(1-15), (29-43), (57-71), (85-99), (113-127), (141-155)) one by one as an antigen on the ELISA plate, adding the antisera obtained from mixture A thereto. When a reaction occurred, it was determined that the peptide in mixture A induced the antibody. The results were calculated as the ratio of the antibody titer one week after the final administration to the antibody titer on the date of the start of administration (S/N ratio).

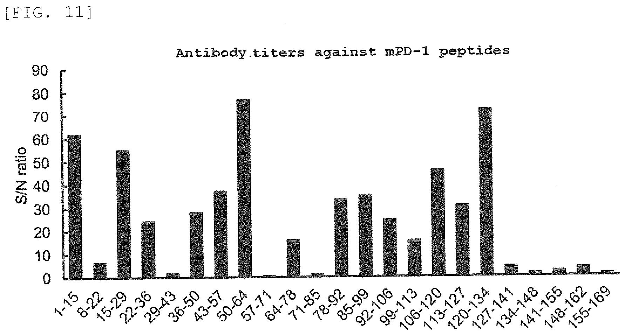

The results are shown in Figure 11. Figure 11 shows that the subcutaneous administration of mPD-1 peptide mixtures combined with an adjuvant resulted in an increase in several anti-mPD-1 peptide antibody titers. From the above, it was demonstrated that, in addition to human TNFα, the combination of the peptide mixtures and the adjuvant was subcutaneously administered to rabbits facilitates to induce antibodies that can recognize the peptides or the derived protein.

The evaluation using flow cytometry was conducted as follows. EL-4 cells (1×10⁶ cells) were mixed with the serum from either the pre-immunization or post-immunization stage (diluted at 10-fold) and the mixture was incubated at 37°C for 1 hour. After washing with PBS, the Cy3-labeled anti-rabbit IgG antibody was reacted thereto at 4°C for 20 minutes. After washing with PBS, the Cy3 fluorescence intensity per cell was measured using a flow cytometer (CytoFLEX, Beckman Coulter).

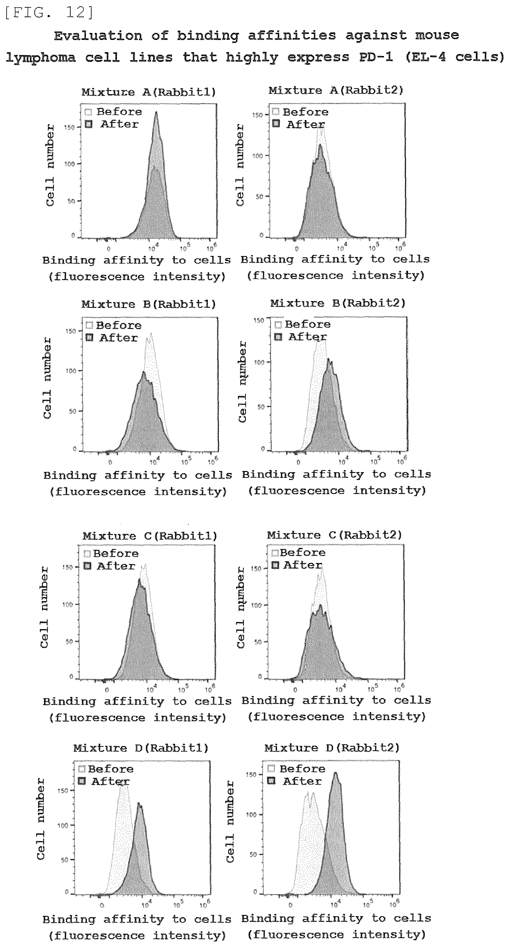

The results of the binding affinity to the EL-4 cells are shown in Figure 12. The antisera of the two rabbits immunized with mixture D among the mPD-1 peptide mixtures to be immunized, significantly increased in the binding affinity to the EL-4 cells that highly expressed PD-1 protein, compared to the pre-immunization. From the above, it was demonstrated that the immunization with the mPD-1 peptide mixtures facilitated to induce antibodies that can recognize the derived protein, PD-1.

### Example 12

### Suppression effect due to immunization with mouse PD-1 peptide mixtures on tumor growth

The suppression effect on tumor growth in the tumor-bearing model mouse was conducted as follows. Emulsions of each mixture at 20 µg/mouse mixed in equal amounts with complete/incomplete Freund's adjuvant were administered subcutaneously once at two-week intervals in a total of six times into the tumor-bearing model mouse. At the time of the fourth administration, blood was collected, and after confirming the increase in antibody titers against mixture A3, B3, C3, or D3, the B16 mouse melanoma cells (2×10⁶ cells/mouse) were subcutaneously transplanted. By observing the subsequent tumor growth, the suppression effect due to immunization with mPD-1 peptide mixtures on tumor growth was evaluated.

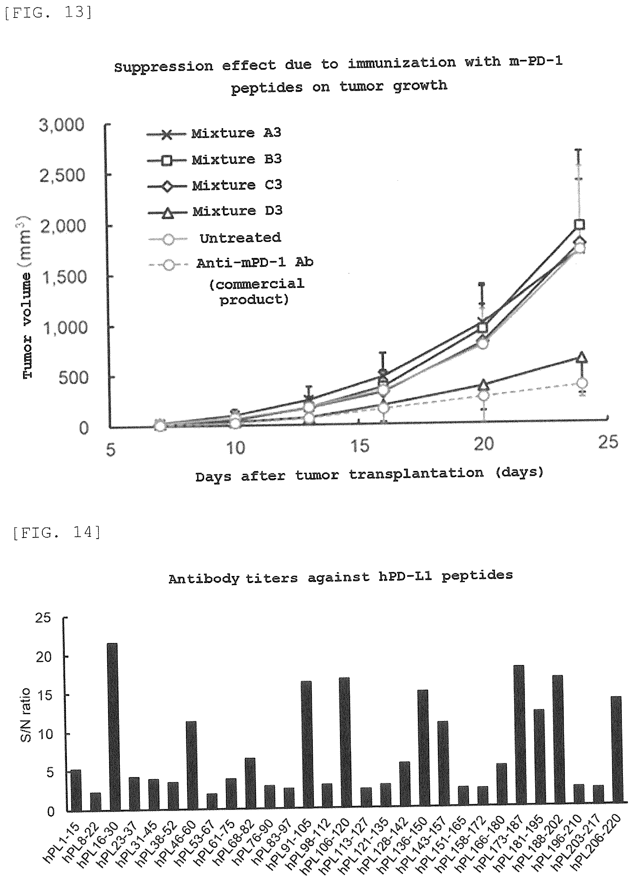

The tumor growth progression in the tumor-bearing model mouse is shown in Figure 13. It was shown that the tumor growth of cancer-bearing mice immunized with mixture D3 among the mPD-1 peptide mixtures to be immunized was suppressed to a level comparable to that of the commercially available anti-mPD-1 monoclonal antibody. From the above, it was demonstrated that the immunization with the mPD-1 peptide mixtures facilitated to induce antibodies capable of recognizing PD-1 expressed on cytotoxic T cells, thereby enhancing the tumor growth suppression activity.

### Example 13

### Measurement of antibody titers of antisera obtained from subcutaneous immunization with human PD-L1 peptide mixtures

Human PD-L1 peptide (hPD-L1 peptide) mixtures were prepared by weighing each hPD-1 peptide to achieve the composition shown in Table 18 and mixing them in equal amounts (weight ratio).

**Table 18: Composition of Various Mixtures of hPD-L1 Peptides**

| [Table 18] | | | |
|---|---|---|---|
| mixture A4 | mixture B4 | mixture C4 | mixture D4 |
| hPD-L1(1-15) FTVTVPKDLYVVEY G (SEQ ID NO:88) | hPD-L1(16-30) SNMTIECKFPVEKQ L (SEQ ID NO:96) | hPD-L1(8-22) DLYVVEYGSNMTIE C (SEQ ID NO:103) | hPD-L1(23-37) KFPVEKQLDLAALI V (SEQ ID NO:110) |
| hPD-L1(31-45) DLAALIVYWEMEDK N (SEQ ID NO:89) | hPD-L1(46-60) IIQFVHGEEDLKVQ H (SEQ ID NO:97) | hPD-L1(38-52) YWEMEDKNIIQFVH G (SEQ ID NO:104) | hPD-L1(53-67) EEDLKVQHSSYRQR A (SEQ ID NO:111) |
| hPD-L1(61-75) SSYRQRARLLKDQL S (SEQ ID NO:90) | hPD-L1(76-90) LGNAALQITDVKLQ D (SEQ ID NO:98) | hPD-L1(68-82) RLLKDQLSLGNAAL Q (SEQ ID NO:105) | hPD-L1(83-97) ITDVKLQDAGVYRC M (SEQ ID NO:112) |
| hPD-L1(91-105) AGVYRCMISYGGAD Y (SEQ ID NO:91) | hPD-L1(106-120 ) KRITVKVNAPYNKI N (SEQ ID NO:99) | hPD-L1(98-112) ISYGGADYKRITVK V (SEQ ID NO:106) | hPD-L1(113-127 ) NAPYNKINQRILVV D (SEQ ID NO:113) |
| hPD-L1(121-135 ) QRILVVDPVTSEHE L (SEQ ID NO:92) | hPD-L1(136-150 ) TCQAEGYPKAEVIW T (SEQ ID NO:100) | hPD-L1(128-142 ) PVTSEHELTCQAEG Y (SEQ ID NO:107) | hPD-L1(143-157 ) PKAEVIWTSSDHQV L (SEQ ID NO:114) |
| hPD-L1(151-165 ) SSDHQVLSGKTTTT N (SEQ ID NO:93) | hPD-L1(166-180 ) SKREEEKLFNVTSTL R (SEQ ID NO:101) | hPD-L1(158-172 ) SGKTTTTNSKREEK L (SEQ ID NO:108) | hPD-L1(173-187 ) FNVTSTLRINTTTN E (SEQ ID NO:115) |
| hPD L1(181 195 ) INTTTNEIFYCTFR R (SEQ ID NO:94) | hPD-L1(196-210 ) LDPEENHTAELVIP E (SEQ ID NO:102) | hPD-L1(188-202 ) IFYCTFRRLDPEEN H (SEQ ID NO:109) | hPD-L1(203-217 ) TAELVIPELPLAHP P (SEQ ID NO:116) |
| hPD-L1(206-220 ) LVIPELPLAHPPNE R (SEQ ID NO:95) | | | |

Emulsions of each mixture at 0.7 mg/rabbit, or at 0.8 mg/rabbit (0.1 mg of each peptide per rabbit) mixed in equal amounts with complete/incomplete Freund's adjuvant were administered subcutaneously once at two-week intervals in a total of four times into rabbits. Blood samples were collected on the date of the start of administration and one week after the final administration, obtaining the antisera in a toral number of 4 against mixtures A4, B4, C4, and D4. The antibody titers against each hPD-1 peptide were measured using the ELISA. The experimental method involved sold-phasing each peptide shown in Table 18 one by one as an antigen on the ELISA plate, adding the antisera obtained from mixtures A4, B4, C4, or D4 thereto. When a reaction occurred, it was determined that the peptide in the mixtures induced the antibody. The results were calculated as the ratio of the antibody titer one week after the final administration to the antibody titer on the date of the start of administration (S/N ratio).

The results are shown in Figure 14. Figure 14 shows that the subcutaneous administration of human PD-1 peptide mixtures combined with an adjuvant resulted in an increase in several anti-human PD-1 peptide antibody titers. From the above, it was demonstrated that the combination of the peptide mixtures and the adjuvant was subcutaneously administered facilitated to induce antibodies that can recognize human PD-L1 peptides.

### Example 14

### Measurement of antibody titers of antisera obtained from subcutaneous immunization with human TNFα peptides having different amino acid sequences alone

In order to examine the elongation of the peptide chain in a method to enhance an activity to induce the antibody against TNF, the chain of the human TNFα peptide that induced the antibody in Example 8 was elongated. The antibody titer of the antiserum obtained from the administration of the human TNFα peptide alone with an elongated amino acid sequence was measured.

Specifically, referring to hTNFα (143-157) that was the C-terminal 15 amino acid sequence, hTNFα (138-157), hTNFα (133-157), and hTNFα (128-157), which extended in one direction, were synthesized (Table 19), and the antibody titers of the antisera obtained from the administrations of the single peptide were measured. In this regard, the reason for selecting the human TNFα (143-157) as a reference peptide, which was a C-terminal peptide with low induction of anti-TNF-α antibody, was as follows. In view of the fact that the C-terminus of the TNF-α trimeric higher-order structure was exposed to the exterior in solution, and that the C-terminus was also responsible for binding to the TNF-α receptor, this peptide was considered to be an important part, and it was expanded N-terminally by five amino acids.

**Table 19: Synthesis of Human TNFα C-terminal Peptides with Different Amino Acid Numbers**

| [Table 19] | | | |
|---|---|---|---|
| hTNFα Peptides | Amino acid sequences | Number of Amino acid s | SEQ ID NOs |
| hTNFα (143-157) | DFAESGQVYFGIIA L | 15AA | SEQ ID NO: 53 |
| hTNFα(138-157) | RPDYLDFAESGQVY FGIIAL | 20AA | SEQ ID NO: 117 |
| hTNFα (133-157) | SAEINRPDYLDFAE SGQVYFGIIAL | 25AA | SEQ ID NO: 118 |
| hTNFα (128-157) | KGDRLSAEINRPDY LDFAESGQVYFGII AL | 30AA | SEQ ID NO: 119 |

Emulsions of hTNFα(143-157), hTNFα(138-157), hTNFα(133-157), or hTNFα(128-157) alone at 1 mg/rabbit mixed in equal amounts with complete/incomplete Freund's adjuvant were administered subcutaneously once at two-week intervals in a total of six times into rabbits. Blood samples were collected on the date of the start of administration and one week after the final administration, obtaining the antisera. The antibody titers against human TNFα were measured using the ELISA. The results were calculated by converting them to the antibody concentration of Humira (registered trademark), which shows the binding capacity equivalent to that of the antibody titer of the antiserum.

The results are shown in Figure 15. Although an increase in antibody titer was observed for hTNFα (143-157) with the short amino acid sequence, by elongating the amino acid sequence, the antibody titer increased further, a higher antibody titer was provided for hTNFα (128-157) with an amino acid sequence of 30AA. From the above, it was demonstrated that, in the immunization with the peptide alone, increasing the length of the amino acid sequence of the immunogenic peptide significantly enhanced the antibody titer against the derived protein.

Multiple human TNFα peptides of 30 amino acids in length (30AA) with high antibody induction ability were synthesized (Table 20), and the antibody titers of the antisera obtained from single administration were measured.

**Table 20: Synthesis of Human TNFα Peptides with Amino Acid Sequence of 30AA**

| [Table 20] | | | |
|---|---|---|---|
| hTNFα peptides | Amino acid sequences | Number of Amino acid | SEQ ID NOs |
| hTNFα(1-30) | VRSSSRTPSDKPVA HVVANPQAEGQLQW LN | 30AA | SEQ ID NO:120 |
| hTNFα (8-37) | PSDKPVAHVVANPQ AEGQLQWLNRRANA LL | 30AA | SEQ ID NO:121 |
| hTNFα (31-60) | RRANALLANGVELR DNQLVVPSEGLYLI YS | 30AA | SEQ ID NO:122 |
| hTNFα (38-67) | ANGVELRDNQLVVP SEGLYLIYSQVLFK GQ | 30AA | SEQ ID NO:123 |
| hTNFα(91-120) | VNLLSAIKSPCQRE TPEGAEAKPWYEPI YL | 30AA | SEQ ID NO:124 |
| hTNFα(121-150) | GGVFQLEKGDRLSA EINRPDYLDFAESG QV | 30AA | SEQ ID NO:125 |
| hTNFα(128-157) | KGDRLSAEINRPDY LDFAESGQVYFGII AL | 30AA | SEQ ID NO:126 |

Emulsion of each human TNFα peptide shown in Table 20 alone at 20 mg/mouse mixed in equal amounts with complete/incomplete Freund's adjuvant were administered subcutaneously one at two-week intervals in total of 6 times into BALB/c mice (6-week-old, female). Blood samples were collected one week after the final administration, and the antiserum was obtained. The antibody titer against human TNFα was measured using the ELISA. The result was calculated by converting it to the antibody concentration of Humira (registered trademark), which shows a binding capacity equivalent to that of the antibody titer of the antiserum.

The results are shown in Figure 16. An increase in the titer of anti-human TNFα antibodies was observed in most human TNFα peptide sequences that were immunized, and particularly a significant increase in antibody titer was noted in the immunizations of hTNFα(8-37), hTNFα(1-30), and hTNFα(91-120). From the above, it was demonstrated that the subcutaneous administration of the 30AA peptide of human TNFα alone combined with an adjuvant facilitated to strongly induce antibodies that can recognize the human TNFα protein.

### Example 15

### Measurement of antibody titers of antiserum obtained from subcutaneous immunization with inactivated human TNFα protein

Based on the results of Example 14, it was recognized that the immunization with human TNFα peptide having the longer amino acid sequence was important for inducing antibodies, and therefore, antibody induction through subcutaneous immunization was conducted with the full-length human TNFα protein (157AA), extending the amino acid sequence to its maximum. Since human TNFα protein has a strong inflammatory induction ability, an inactivated mutant human TNFα protein (hTNFα(V91D)) was prepared.

### hTNFα (V91D) (157AA) :

VRSSSRTPSDKPVAHVVANPQAEGQLQWLNRRANALLANGVELRDNQLVVPSEGLYLIY

SQVLFKGQGCPSTHVLLTHTISRIAVSYQTKDNLLSAIKSPCQRETPEGAEAKPWYEPI

YLGGVFQLEKGDRLSAEINRPDYLDFAESGQVYFGIIAL (SEQ ID NO: 127)

Human TNFα (V91D) was prepared by designing a plasmid DNA expressing human TNFα (V91D), introducing the same into Chinese hamster ovary cells (CHO cells) to express human TNFα (V91D), which was then purified. Emulsion of human TNFα (V91D) alone at 20 µg/mouse mixed in equal amounts with complete/incomplete Freund's adjuvant was administered subcutaneously a total of four times at two-week intervals to BALB/c mice (6 weeks old, female). Blood samples were collected one week after the final administration, and the antiserum was obtained. The antibody titer against the wild-type human TNFα was measured using ELISA.

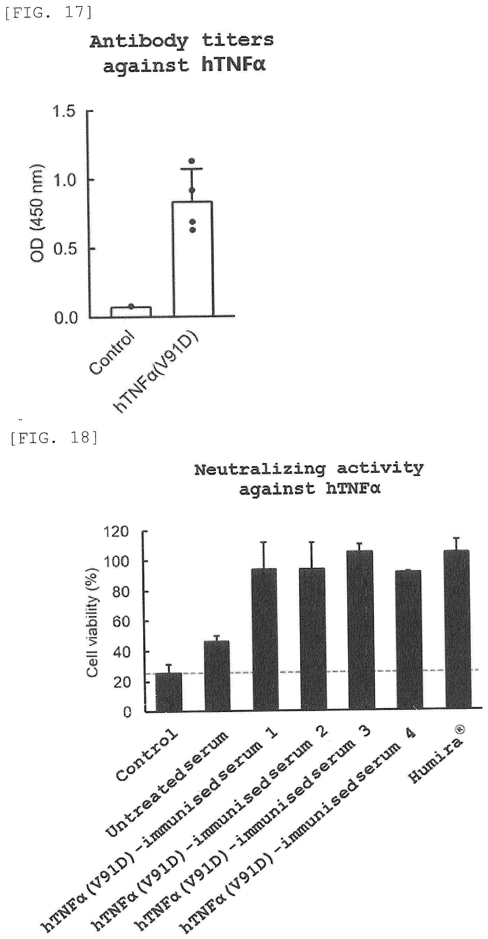

The results are shown in Figure 17. It was demonstrated that subcutaneous immunization with the inactivated human TNFα (V91D) induced antibodies that recognize the wild-type human TNFα.

The evaluation of an activity neutralizing human TNFα of the antiserum obtained from the immunization with the human TNFα (V91D) was conducted as follows. L929 mouse fibroblasts that were highly sensitive to TNFα were seeded in a 96-well plate (2×10⁴ cells/well), and the plate was cultured for 24 hours. After mixing the antiserum (10-fold dilution) or Humira (trademark) with human TNFα (3 ng/mL) and allowing the same to react for 1 hour, the human TNFα mixture and actinomycin D (2.7 µg/mL) were added to the cells and the plate was cultured for 24 hours. The subsequent cell viability was measured using the MTT assay. The result was calculated as a survival rate in which the survival of the cells without adding human TNFα was set at 100%.

The results are shown in Figure 18. The control group and the untreated serum addition group showed that the addition of human TNFα resulted in a decrease in cell viability to 25% through 45%, whereas the human TNFα (V91D) immunization serum addition group showed nearly 100% cell viability, which was a level of inhibition of the cytotoxicity activity (neutralizing activity) comparable to that of Humira (registered trademark). From the above, it was demonstrated that antibodies induced by subcutaneous immunization with the inactivated human TNFα mutant facilitated to inhibit the cytotoxic activity of the wild-type human TNFα.

### Example 17

### Blood clearance effect of target protein by the partial peptide antiserum

The antiserum recovered by immunizing with the human TNFα peptides of 30AA (hTNFα(8-37), hTNFα(91-120), or hTNFα(128-157)) identified in Example 14, which showed high antibody induction were used to evaluate the effect on the clearance of human TNFα in blood. The emulsion of hTNFα(8-37), hTNFα(91-120), or hTNFα(128-157) alone at 1 mg/rabbit mixed in equal amounts with complete/incomplete Freund's adjuvant were administered subcutaneously once at two-week intervals in a total of four times into rabbits. Blood samples were collected one week after the final administration, and the antiserum was obtained. The serum collected from the antiserum or the serum from the untreated rabbit was purified using a Protein G column to provide polyclonal antibodies. The polyclonal antibodies were administered intravenously at a total antibody amount of 1 mg/mouse to BALB/c mice (6 weeks old, male), and then 15 minutes later, human TNFα was administered intraperitoneally at a dose of 1 µg/mouse. Thirty minutes after the administration, the serum was collected, and the concentration of human TNFα in the serum was measured using ELISA. When the induced anti-human TNFα serum had a neutralizing activity, it would bind to and eliminate the human TNFα that had transitioned from the peritoneal cavity into the bloodstream. Therefore, it was assumed that the concentration of the administered human TNFα in blood should be relatively lower compared to the presence of polyclonal antibodies derived from untreated serum (control polyclonal antibodies).

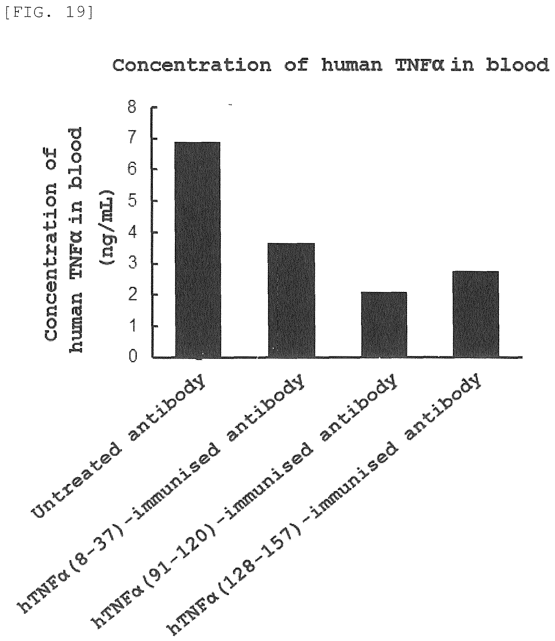

The results are shown in Figure 19. The group administered with the polyclonal antibodies derived from untreated serum showed that the peak concentration of human TNFα in the serum was 6.9 ng/mL, whereas the group administered with the polyclonal antibodies obtained by immunizing with hTNFα(8-37) showed a concentration of 3.7 ng/mL, the group immunized with hTNFα(91-120) showed 2.1 ng/mL, and the group immunized with hTNFα(128-157) showed 2.8 ng/mL, demonstrating that the concentration of human TNFα in the serum decreased in the presence of polyclonal antibodies in post-immunization. From the above, it was demonstrated that the polyclonal antibodies obtained by immunizing with the human TNFα peptide of 30AA promoted the clearance of human TNFα in the blood and exhibited a neutralizing activity.

The present invention may further encompass the following embodiments.

### <Methods for preparing partial peptides having abilities to induce antibodies against target proteins>

[101] A method for preparing a partial peptide within a target protein, which induces an antibody specific to the target protein, which comprises:
1) providing a plurality of partial peptides of 5-30 amino acid residues within the amino acid sequence of the target protein, wherein each partial peptide may overlap with an adjacent different partial peptide by a specific number of amino acid residues at its N-terminus or C-terminus, and wherein the plurality of the partial peptides covers the entire region of the amino acid sequence of the target protein;
2) making a mixture containing the 4 to 12 partial peptides obtained;
3) immunizing non-human mammals by use of the obtained mixture;
4) determining whether the mixture induces any antibody specific to the target protein by the immunization; and
5) identifying the partial peptide that induces the specific antibody from the mixture that has been determined to induce the antibody.

[102] The method according to [101], which further comprises;
6) a step of adding an additional sequence to the identified partial peptide.

[103] The method according to any one of [101] to [104], wherein the non-human mammal is a mouse, rabbit, or monkey in step 3).

[104] The method according to [103], wherein step 6) comprises adding the additional sequence to make the partial peptide 30 amino acids in length.

[105] The method according to [102], wherein step 6) comprises adding the additional sequence to make the identified partial peptide 50 amino acids in length or less.

[106] The method according to any one of [101] to [105], wherein the partial peptide contained in the mixture is not chemically bonded to any carrier protein.

### <Partial peptides having abilities to induce antibodies against target proteins>

[107] A partial peptide within a target protein identified by the method for preparation according to [1], which induces an antibody specific to the target protein, or a mutant partial peptide having one or several substitutions, deletions, or additions of amino acid residues within the amino acid sequence of the partial peptide, and having an equivalent activity to the partial peptide.
[108] The partial peptide or the mutant partial peptide according to [107], wherein the partial peptide induces an antibody specifically binding to human TNFα, and has an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCORETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), and wherein the mutant partial peptide has one or several substitutions, deletions, or additions of amino acid residues within the amino acid sequence of the partial peptide, and has an equivalent activity to the partial peptide.
[109] The partial peptide or the mutant partial peptide according to [107], wherein the partial peptide induces an antibody specifically binding to human β-amyloid peptide, and has an amino acid sequence of SGYEVHHQKLVFFA (SEQ ID NO: 50), and wherein the mutant partial peptide has one or several substitutions, deletions, or additions of amino acid residues within the amino acid sequence of the partial peptide, and has an equivalent activity to the partial peptide.
[110] The partial peptide or the mutant partial peptide according to [107], wherein the partial peptide induces an antibody specifically binding to human PD-L1, and has an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), and wherein the mutant partial peptide has one or several substitutions, deletions, or additions of amino acid residues within the amino acid sequence of the partial peptide, and has an equivalent activity to the partial peptide.
[111] The partial peptide or the mutant partial peptide according to [107], wherein the partial peptide induces an antibody specifically binding to human PD-1, and has an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), and wherein the mutant partial peptide has one or several substitutions, deletions, or additions of amino acid residues within the amino acid sequence of the partial peptide, and has an equivalent activity to the partial peptide.
[112] The partial peptide or the mutant partial peptide according to [7], wherein the partial peptide induces an antibody specifically binding to mPD-1, and has an amino acid sequence selected from GWLLEVPNGPWRSLT (SEQ ID NO: 55), ATFTCSLSNWSEDLM (SEQ ID NO: 56), KQAAFCNGLSQPVQD (SEQ ID NO: 57), and FHMNILDTRRNDSGI (SEQ ID NO: 58), and wherein the mutant partial peptide has one or several substitutions, deletions, or additions of amino acid residues within the amino acid sequence of the partial peptide, and has an equivalent activity to the partial peptide.

### <Peptide vaccines>

[113] A vaccine for preventing and/or treating a disease related to a target protein, which comprises a partial peptide within the target protein identified by the method for preparation according to [101], which induces an antibody specific to the target protein, or a mutant partial peptide.
[114] The vaccine according to [113], which comprises a plurality of the partial peptides and/or the mutant partial peptides.
[115] The vaccine according to [113] or [114] for preventing and/or treating a disease related to human TNFα, wherein the partial peptide induces an antibody specifically binding to human TNFα, and has an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCQRETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), and wherein the mutant partial peptide has one or several substitutions, deletions, or additions of amino acid residues within the amino acid sequence of the partial peptide, and has an equivalent activity to the partial peptide.
[116] The vaccine according to [113] or [114] for preventing and/or treating a disease related to human β-amyloid peptide, wherein the partial peptide induces an antibody specifically binding to human β-amyloid peptide, and has an amino acid sequence SGYEVHHQKLVFFA (SEQ ID NO: 50), and wherein the mutant partial peptide has one or several substitutions, deletions, or additions of amino acid residues within the amino acid sequence of the partial peptide, and has an equivalent activity to the partial peptide.
[117] The vaccine according to [113] or [114] for preventing and/or treating a disease related to human PD-L1 protein, wherein the partial peptide induces an antibody specifically binding to human PD-L1 protein, and has an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), and wherein the mutant partial peptide has one or several substitutions, deletions, or additions of amino acid residues within the amino acid sequence of the partial peptide, and has an equivalent activity to the partial peptide.
[118] The vaccine according to [113] or [114] for preventing and/or treating a disease related to human PD-1 protein, wherein the partial peptide induces an antibody specifically binding to human PD-1 protein, and has an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), and wherein the mutant partial peptide has one or several substitutions, deletions, or additions of amino acid residues within the amino acid sequence of the partial peptide, and has an equivalent activity to the partial peptide.

### <RNA vaccines>

[119] A vaccine for preventing and/or treating a disease related to a target protein, which comprises mRNA coding for a partial peptide within the target protein identified by the method for preparation according to [101], which induces an antibody specific to the target protein, or a mutant partial peptide.
[120] The vaccine according to [119], which comprises a plurality of the mRNAs coding for the partial peptides and/or the mutant partial peptides.
[121] The vaccine according to [119] or [120] for preventing and/or treating a disease related to human TNFα, wherein the mRNA codes for a partial peptide inducing an antibody specifically binding to human TNFα, and having an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCQRETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), or a mutant partial peptide having one or several substitutions, deletions, or additions of amino acid residues within the amino acid sequence of the partial peptide, and having an equivalent activity to the partial peptide.
[122] The vaccine according to [121], wherein the mRNA is selected from GGGCAGCUCCAGUGGCUGAACCGCCGGGCCAAUGCCCUCCUGGCC (SEQ ID NO: 59), CCCUGCCAGAGGGAGACCCCAGAGGGGGCUGAGGCCAAGCCCUGG (SEQ ID NO: 60), CUCCAGUGGCUGAACCGCCGGGCCAAUGCCCUCCUGGCCAAUGGC (SEQ ID NO: 61), and GACUUUGCCGAGUCUGGGCAGGUCUACUUUGGGAUCAUUGCCCUG (SEQ ID NO: 62).
[123] The vaccine according to [119] or [120] for preventing and/or treating a disease related to β-amyloid peptide, wherein the mRNA codes for a partial peptide inducing an antibody specifically binding to β-amyloid peptide, and having an amino acid sequence SGYEVHHQKLVFFA (SEQ ID NO: 50), or a mutant partial peptide having one or several substitutions, deletions, or additions of amino acid residues within the amino acid sequence of the partial peptide, and having an equivalent activity to the partial peptide.
[124] The vaccine according to [123], wherein the mRNA is UCAGGAUAUGAAGUUCAUCAUCAARARAUUGGUGUUCUUUGCA (SEQ ID NO: 63).
[125] The vaccine according to [119] or [120] for preventing and/or treating a disease related to human PD-L1 protein, wherein the mRNA codes for a partial peptide inducing an antibody specifically binding to human PD-L1 protein, and having an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), or a mutant partial peptide having one or several substitutions, deletions, or additions of amino acid residues within the amino acid sequence of the partial peptide, and having an equivalent activity to the partial peptide.
[126] The vaccine according to [119] or [120] for preventing and/or treating a disease related to human PD-1 protein, wherein the mRNA codes for a partial peptide inducing an antibody specifically binding to human PD-1 protein, and having an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), or a mutant partial peptide having one or several substitutions, deletions, or additions of amino acid residues within the amino acid sequence of the partial peptide, and having an equivalent activity to the partial peptide.

## Claims

1. A method for preparing a partial peptide within a target protein, which induces an antibody specific to the target protein, which comprises:
1) providing a plurality of partial peptides of 5-30 amino acid residues within the amino acid sequence of the target protein, wherein each partial peptide may overlap with an adjacent different partial peptide by a specific number of amino acid residues at its N-terminus or C-terminus, and wherein the plurality of the partial peptides covers the entire region of the amino acid sequence of the target protein;
2) making a mixture containing the 4 to 12 partial peptides obtained;
3) immunizing non-human mammals by use of the obtained mixture;
4) determining whether the mixture induces any antibody specific to the target protein by the immunization; and
5) identifying the partial peptide that induces the specific antibody from the mixture that has been determined to induce the antibody.

2. The method according to claim 1, which further comprises;
6) a step of adding an additional sequence to the identified partial peptide.

3. The method according to claim 2, wherein step 6) comprises adding the additional sequence to make the identified partial peptide 50 amino acids in length or less.

4. The method according to claim 3, wherein step 6) comprises adding the additional sequence to make the partial peptide 30 amino acids in length.

5. The method according to claim 1, wherein the non-human mammal is a mouse, rabbit, or monkey in step 3).

6. The method according to claim 1, wherein the partial peptide contained in the mixture is not chemically bonded to any carrier protein.

7. A partial peptide within a target protein identified by the method for preparation according to claim 1, which induces an antibody specific to the target protein, or a mutant partial peptide having one or several substitutions, deletions, or additions of amino acid residues within the amino acid sequence of the partial peptide, and having an equivalent activity to the partial peptide.

8. The partial peptide or the mutant partial peptide according to claim 7, wherein the partial peptide induces an antibody specifically binding to human TNFα, and has an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCQRETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), and wherein the mutant partial peptide has one or several substitutions, deletions, or additions of amino acid residues within the amino acid sequence of the partial peptide, and has an equivalent activity to the partial peptide.

9. The partial peptide or the mutant partial peptide according to claim 7, wherein the partial peptide induces an antibody specifically binding to human β-amyloid peptide, and has an amino acid sequence of SGYEVHHQKLVFFA (SEQ ID NO: 50), and wherein the mutant partial peptide has one or several substitutions, deletions, or additions of amino acid residues within the amino acid sequence of the partial peptide, and has an equivalent activity to the partial peptide.

10. The partial peptide or the mutant partial peptide according to claim 7, wherein the partial peptide induces an antibody specifically binding to human PD-L1, and has an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), and wherein the mutant partial peptide has one or several substitutions, deletions, or additions of amino acid residues within the amino acid sequence of the partial peptide, and has an equivalent activity to the partial peptide.

11. The partial peptide or the mutant partial peptide according to claim 7, wherein the partial peptide induces an antibody specifically binding to human PD-1, and has an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), and wherein the mutant partial peptide has one or several substitutions, deletions, or additions of amino acid residues within the amino acid sequence of the partial peptide, and has an equivalent activity to the partial peptide.

12. The partial peptide or the mutant partial peptide according to claim 7, wherein the partial peptide induces an antibody specifically binding to mPD-1, and has an amino acid sequence selected from GWLLEVPNGPWRSLT (SEQ ID NO: 55), ATFTCSLSNWSEDLM (SEQ ID NO: 56), KQAAFCNGLSQPVQD (SEQ ID NO: 57), and FHMNILDTRRNDSGI (SEQ ID NO: 58), and wherein the mutant partial peptide has one or several substitutions, deletions, or additions of amino acid residues within the amino acid sequence of the partial peptide, and has an equivalent activity to the partial peptide.

13. A vaccine for preventing and/or treating a disease related to a target protein, which comprises a partial peptide within the target protein identified by the method for preparation according to claim 1, which induces an antibody specific to the target protein, or a mutant partial peptide.

14. The vaccine according to claim 13, which comprises a plurality of the partial peptides and/or the mutant partial peptides.

15. The vaccine according to claim 13 for preventing and/or treating a disease related to human TNFα, wherein the partial peptide induces an antibody specifically binding to human TNFα, and has an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDEPVAHVVANPQAEGQLOQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCORETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), and wherein the mutant partial peptide has one or several substitutions, deletions, or additions of amino acid residues within the amino acid sequence of the partial peptide, and has an equivalent activity to the partial peptide.

16. The vaccine according to claim 13 for preventing and/or treating a disease related to human β-amyloid peptide, wherein the partial peptide induces an antibody specifically binding to human β-amyloid peptide, and has an amino acid sequence SGYEVHHQKLVFFA (SEQ ID NO: 50), and wherein the mutant partial peptide has one or several substitutions, deletions, or additions of amino acid residues within the amino acid sequence of the partial peptide, and has an equivalent activity to the partial peptide.

17. The vaccine according to claim 13 for preventing and/or treating a disease related to human PD-L1 protein, wherein the partial peptide induces an antibody specifically binding to human PD-L1 protein, and has an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), and wherein the mutant partial peptide has one or several substitutions, deletions, or additions of amino acid residues within the amino acid sequence of the partial peptide, and has an equivalent activity to the partial peptide.

18. The vaccine according to claim 13 for preventing and/or treating a disease related to human PD-1 protein, wherein the partial peptide induces an antibody specifically binding to human PD-1 protein, and has an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), and wherein the mutant partial peptide has one or several substitutions, deletions, or additions of amino acid residues within the amino acid sequence of the partial peptide, and has an equivalent activity to the partial peptide.

19. A vaccine for preventing and/or treating a disease related to a target protein, which comprises mRNA coding for a partial peptide within the target protein identified by the method for preparation according to claim 1, which induces an antibody specific to the target protein, or a mutant partial peptide.

20. The vaccine according to claim 19, which comprises a plurality of the mRNAs coding for the partial peptides and/or the mutant partial peptides.

21. The vaccine according to claim 19 for preventing and/or treating a disease related to human TNFα, wherein the mRNA codes for a partial peptide inducing an antibody specifically binding to human TNFα, and having an amino acid sequence selected from GQLQWLNRRANALLA (SEQ ID NO: 14), PCQRETPEGAEAKPW (SEQ ID NO: 20), LQWLNRRANALLANG (SEQ ID NO: 26), DFAESGQVYFGIIAL (SEQ ID NO: 53), VRSSSRTPSDKPVAHVVANPQAEGQLQWLN (SEQ ID NO: 120), PSDKPVAHVVANPQAEGQLQWLNRRANALL (SEQ ID NO: 121), RRANALLANGVELRDNQLVVPSEGLYLIYS (SEQ ID NO: 122), ANGVELRDNQLVVPSEGLYLIYSQVLFKGQ (SEQ ID NO: 123), VNLLSAIKSPCQRETPEGAEAKPWYEPIYL (SEQ ID NO: 124), GGVFQLEKGDRLSAEINRPDYLDFAESGQV (SEQ ID NO: 125), and KGDRLSAEINRPDYLDFAESGQVYFGIIAL (SEQ ID NO: 126), or a mutant partial peptide having one or several substitutions, deletions, or additions of amino acid residues within the amino acid sequence of the partial peptide, and having an equivalent activity to the partial peptide.

22. The vaccine according to claim 21, wherein the mRNA is selected from GGGCAGCUCCAGUGGCUGAACCGCCGGGCCAAUGCCCUCCUGGCC (SEQ ID NO: 59), GCCUGCCAGAGGGAGACCCCAGAGGGGGCUGAGGCCAAGCCCUGG (SEQ ID NO: 60), CUCCAGUGGCUGAACCGCCGGGCCAAUGCCCUCCUGGCCAAUGGC (SEQ ID NO: 61), and GACUUUGCCGAGUCUGGGCAGGUCUACUUUGGGAUCAUUGCCCUG (SEQ ID NO: 62).

23. The vaccine according to claim 19 for preventing and/or treating a disease related to β-amyloid peptide, wherein the mRNA codes for a partial peptide inducing an antibody specifically binding to β-amyloid peptide, and having an amino acid sequence SGYEVHHQKLVFFA (SEQ ID NO: 50), or a mutant partial peptide having one or several substitutions, deletions, or additions of amino acid residues within the amino acid sequence of the partial peptide, and having an equivalent activity to the partial peptide.

24. The vaccine according to claim 23, wherein the mRNA is UCAGGAUAUGAAGUUCAUCAUCAAAAAUUGGUGUUCUUUGCA (SEQ ID NO: 63).

25. The vaccine according to claim 19 for preventing and/or treating a disease related to human PD-L1 protein, wherein the mRNA codes for a partial peptide inducing an antibody specifically binding to human PD-L1 protein, and having an amino acid sequence selected from AGVYRCMISYGGADY (SEQ ID NO: 91), INTTTNEIFYCTFRR (SEQ ID NO: 94), LVIPELPLAHPPNER (SEQ ID NO: 95), SNMTIECKFPVEKQL (SEQ ID NO: 96), IIQFVHGEEDLKVQH (SEQ ID NO: 97), KRITVKVNAPYNKIN (SEQ ID NO: 99), TCQAEGYPKAEVIWT (SEQ ID NO: 100), IFYCTFRRLDPEENH (SEQ ID NO: 109), PKAEVIWTSSDHQVL (SEQ ID NO: 114), and FNVTSTLRINTTTNE (SEQ ID NO: 115), or a mutant partial peptide having one or several substitutions, deletions, or additions of amino acid residues within the amino acid sequence of the partial peptide, and having an equivalent activity to the partial peptide.

26. The vaccine according to claim 19 for preventing and/or treating a disease related to human PD-1 protein, wherein the mRNA codes for a partial peptide inducing an antibody specifically binding to human PD-1 protein, and having an amino acid sequence selected from ESFVLNWYRMSPSNQ (SEQ ID NO: 80), YLCGAISLAPKAQIK (SEQ ID NO: 82), EGDNATFTCSFSNTS (SEQ ID NO: 84), and FHMSVVRARRNDSGT (SEQ ID NO: 86), or a mutant partial peptide having one or several substitutions, deletions, or additions of amino acid residues within the amino acid sequence of the partial peptide, and having an equivalent activity to the partial peptide.
